# EUROPEAN PATENT SPECIFICATION

(11) **EP 1 833 801 B1**
(45) Date of publication and mention of the grant of the patent: **04.06.2008**
(21) Application number: 05823669.6
(22) Date of filing: 22.12.2005
(51) Int. Cl.: C07D 233/22

(54) **INTERMEDIATE COMPOUNDS FOR THE PREPARATION OF ANGIOTENSIN II RECEPTOR ANTAGONISTS**
ZWISCHENPRODUKTE FÜR DIE HERSTELLUNG VON ANTAGONISTEN DES ANGIOTENSIN-II-REZEPTORS
COMPOSES INTERMEDIAIRES POUR LA PREPARATION D'ANTAGONISTES DU RECEPTEUR DE L'ANGIOTENSINE II

(30) Priority: 22.12.2004 EP 04106861
(43) Date of publication of application: 19.09.2007
(73) Proprietor: ALGRY QUIMICA, S.L, 28036 Madrid (ES)
(72) Inventor: DÍAZ MARTÍNEZ, Adolfo, E-28903 Getafe (madrid) (ES); RAFECAS JANÉ , Llorenç, E-43712 Llorenç Del Penedès (tarragona) (ES); RIERA ESCALÉ , Antoni, E-08005 Barcelona (ES); PASTÓ AGUILÀ, Mireia, E-08028 Barcelona (ES); ÉCIJA QUERALT, Marta, E-08027 Barcelona (ES)
(74) Representative: Barlocci, Anna
(86) International application number: PCT/EP2005/057102
(87) International publication number: WO 2006/067215

(56) References cited:
- EP-A- 0 459 136
- EP-A- 0 994 881
- EP-A- 1 384 717
- WO-A-93/10106
- WO-A-20/04065383
- MIYAURA N ET AL: "THE PALLADIUM-CATALYZED CROSS-COUPLING REACTION OF PHENYLBORONIC ACID WITH HALOARENES IN THE PRESENCE OF BASES" SYNTHETIC COMMUNICATIONS, MARCEL DEKKER, INC., BASEL, CH, vol. 11, no. 7, 1981, pages 513-519, XP009023474 ISSN: 0039-7911

## Description

The present invention relates to new substituted heterocyclic methylphenyl boronic acids and their derivatives, which are intermediates useful for the preparation of Angiotensin II (A-II) receptor antagonists. It relates also to their preparation processes, as well as to a process for the preparation of A-II receptor antagonists from such intermediates.

### BACKGROUND ART

Angiotensin II is a powerful vasopressor and a stimulator of aldosterone secretion by the adrenal cortex. By its vasopressor action, it raises blood pressure and diminishes fluid loss in the kidney by restricting flow.

A-II receptor antagonists appear to be as effective as Angiotensin converting enzyme (ACE) inhibitors in the treatment of hypertension and other pathologic processes that involve the renin-angiotensin-system (RAS) and may offer an alternative to those patients who cannot tolerate ACE inhibitors because of their side effects. They exert its activity blocking the binding of Angiotensin II to the Angiotensin II type 1 (AT₁) receptor, the receptor that mediates the cardiovascular effects of Angiotensin II.

Substituted imidazole derivatives having a biphenyltetrazolyl moiety have proven to be potent ligands for A-II receptors and a number of them are used mainly for the treatment of hypertension. Between these A-II receptors antagonists are losartan, 2-n-butyl-4-chloro-1-{[2'-(1H-tetrazol-5-yl)-1,1'-biphenyl-4-yl]methyl}-1H-imidazole-5-methanol; irbesartan, 2-n-butyl-3-{[2'-(1H-tetrazol-5-yl)-1,1'-biphenyl-4-yl]methyl}-1,3-diazaspiro [4.4]non-1-en-4-one; and candesartan, 2-ethoxy-1-{[2'-(1H-tetrazol-5-yl)-1,1'-biphenyl-4-yl]methyl}-benzimidazole-7-carboxylic acid.

Several methods for preparing such compounds have been described in the art. Most of them involve a coupling reaction between a compound containing the imidazole moiety with a compound containing the biphenyl moiety (cf. e.g. EP 253.310-A, EP 454.511-A and EP 459.136-A).

Such type of compounds has also been prepared by processes which involve the coupling of the phenyl moieties. Thus, in EP 994.881-A a preparation process of 2-substituted-1-(tetrazol-5-yl)benzenes by coupling a suitable bromophenylderivative with an ortho-methalated (tetrazol-5-yl)benzene is described, as well as the preparation of Angiotensin II antagonists from such intermediates. In EP 643.704-A a preparation process of losartan by coupling 2-(2'-triphenylmethyl-2'-H-tetrazol-5'-yl)phenylboronic acid with 1-bromo-4-(2'-butyl-4'-chloro-5'-hydroxymethylimidazole-1'H-1'yl)methylbenzene in a very specific reaction conditions is also described.

Thus, from what is known in the art it is derived that the provision of an efficient alternative process for the preparation of substituted heterocyclic derivatives having a biphenyltetrazolyl moiety would be of great interest in industry.

### SUMMARY OF THE INVENTION

Inventors have found a new preparation process of substituted heterocyclic derivatives acting as A-II receptor antagonists from new substituted heterocyclic methylphenyl boronic acids and their derivatives, which proceeds with high yields and high regioselectivity.

Thus, according to an aspect of the present invention, there is provided a compound of formula (II), 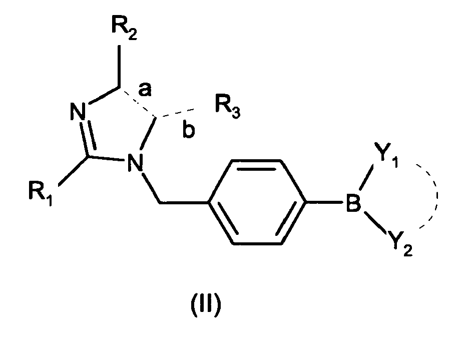 where Y₁ and Y₂ are each independently selected from the group consisting of hydroxy, (C₁-C₄)-alcoxy and phenoxy, the latter optionally substituted by a (C₁-C₄)-alcoxy, (C₁-C₄)-alkyl or an halogen group; or alternatively Y₁ and Y₂ can be taken together with the boron atom to form a cyclic structure selected from the following ones, 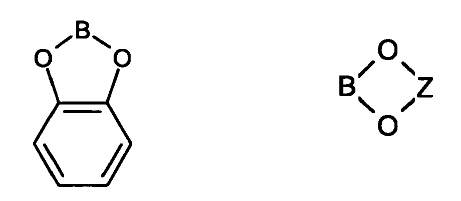 where: Z is selected from the group consisting of (CH₂)ₙ, (CH₂)ᵣCRᵤRᵥ(CH₂)ₛ and CRᵤRᵥ(CH₂)ₜCRᵤRᵥ; n is an integer from 2 to 4; r and s are integers from 0 to 4 with the condition that r and s are not both 0; t is an integer from 0 to 1, and Rᵤ and Rᵥ are each independently selected from the group consisting of H, (C₁-C₄)-alkyl, phenyl and mono- or di- substituted phenyl, the substituents being halogen, (C₁-C₄)-alkyl and (C₁-C₄)-alkoxy;
a and b are single or double bonds; R₁ is a radical selected from (C₁-C₄)-alkyl and (C₁-C₄)-alkoxy; R₂ is Cl, or alternatively R₂ forms a spiro-fused ring from 4 to 6 carbon atoms with the carbon atom of the heterocyclic ring; R₃ is O or a radical selected from the group consisting of hydroxymethyl and formyl, which are optionally protected; or alternatively, R₂ and R₃ are bound together forming with the heterocyclic ring a fused benzene ring substituted by a radical selected from the group consisting of (C₁-C₆)-alkoxycarbonyl and 1-{[(cyclohexyloxy)carbonyl]oxy}ethoxycarbonyl; with the condition that:
(i) when a is a single bond, b is a double bond, R₂ forms a spiro-fused ring from 4 to 6 carbon atoms with the carbon atom of the heterocyclic ring; and R₃ is O; and
(ii) when a is a double bond, b is a single bond, R₂ is Cl, and R₃ is a radical selected from the group consisting of hydroxymethyl and formyl, which are optionally protected; or alternatively, R₂ and R₃ are bound together forming with the heterocyclic ring a fused benzene ring, substituted by a radical selected from the group consisting of (C₁-C₆)-alkoxycarbonyl and 1-{[(cyclohexyloxy)carbonyl]oxy}ethoxycarbonyl.

The invention is also related to a preparation process of a compound of formula (II), comprising a reaction of a compound of formula (III), 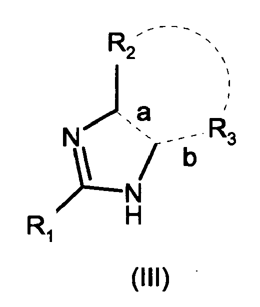 with a boron compound of formula (IV) 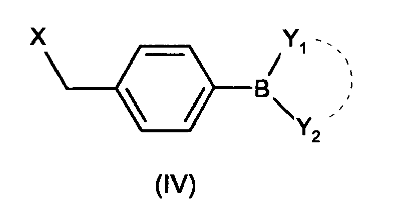 in the presence of a base and an appropriate solvent, where X is a leaving group such as chlorine, bromine, iodine, methanesulfonyloxy, toluensulfonyloxy, benzenesulfonyloxy or trifluoromethanesulfonyloxy; and each of R₁, R₂ ,R₃, Y₁ and Y₂ is a group as defined above, or an intermediate or protected form thereof which can be transformed to such a group, and thereafter as necessary transforming said intermediate or protected forms of the R₁, R₂ ,R₃, Y₁ and Y₂ groups to R₁, R₂ ,R₃, Y₁ and Y₂ as defined above.

Compounds of formula (II), wherein R₁ is a (C₁-C₄)-alkoxy, a is a double bond, and R₂ and R₃ are bound together forming with the heterocyclic ring a fused benzene ring substituted by a radical selected from the group consisting of (C₁-C₆)-alkoxycarbonyl and 1-{[(cyclohexyloxy)carbonyl]oxy}ethoxycarbonyl, represented by the formula (II'), can also be prepared by a process comprising the steps of:
(a) reacting a compound of formula (V), 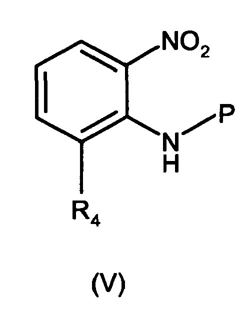 with the boron compound of formula (IV) defined above in the presence of a base and an appropriate solvent; followed by the cleavage of the protective amino group P and the reduction of the nitro group to yield a compound of formula (VI); and 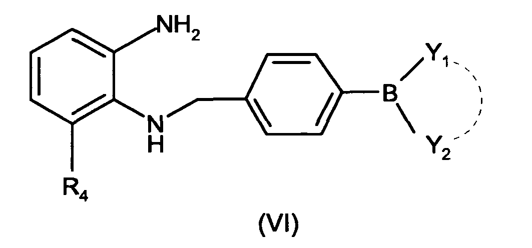
(b) treating the compound (VI) with alkyl orthocarbonate or a carbonylating agent, to give a compound of formula (II'); 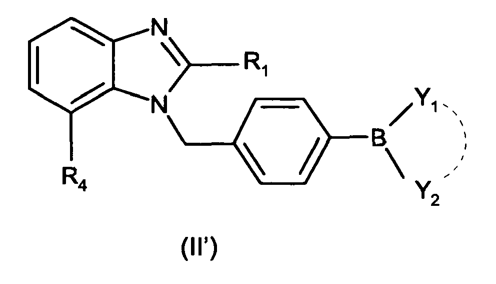 In formula (II'), R₄ is a radical selected from (C₁-C₆)-alkoxycarbonyl and 1-{[(cyclohexyloxy)carbonyl]oxy}ethoxycarbonyl and each of R₄, R₁, Y₁ and Y₂ is a group as defined above or an intermediate or protected form thereof which can be transformed to such a group, and thereafter as necessary transforming said intermediate or protected forms of the R₄, R₁, Y₁ and Y₂ groups to R₄, R₁, Y₁ and Y₂ groups as defined above.

The invention is also related to a preparation process of a compound of formula (1), 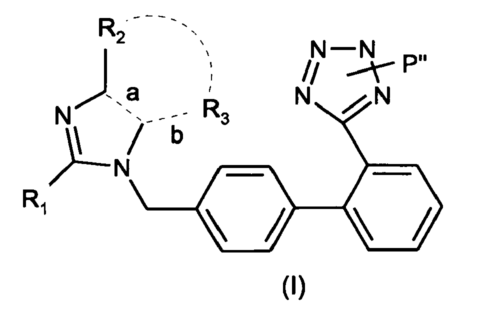 where a and b are single or double bonds; P" is H or a protective group P'; R₁ is a radical selected from (C₁-C₄)-alkyl and (C₁-C₄)-alkoxy; R₂ is Cl, or alternatively R₂ forms a spiro-fused ring from 4 to 6 carbon atoms with the carbon atom of the heterocyclic ring, R₃ is O or a radical selected from the group consisting of hydroxymethyl and formyl, which are optionally protected; or alternatively, R₂ and R₃ are bound together forming with the heterocyclic ring a fused benzene ring substituted by a radical selected from the group consisting of carboxyl, (C₁-C₆)-alkoxycarbonyl and
1-{[(cyclohexyloxy)carbonyl]oxy}-ethoxycarbonyl; with the condition that: (i) when a is a single bond, b is a double bond, R₂ forms a spiro-fused ring from 4 to 6 carbon atoms with the carbon atom of the heterocyclic ring and R₃ is O; and (ii) when a is a double bond, b is a single bond, R₂ is Cl; and R₃ a radical selected from the group consisting of hydroxymethyl and formyl, which are optionally protected; or alternatively, R₂ and R₃ are bound together forming with the heterocyclic ring a fused benzene ring, substituted by a radical selected from the group consisting of carboxyl, (C₁-C₆)-alkoxycarbonyl and 1-{[(cyclohexyloxy)carbonyl]oxy}ethoxycarbonyl; said process comprises a coupling reaction of a compound of formula (II), as defined above, 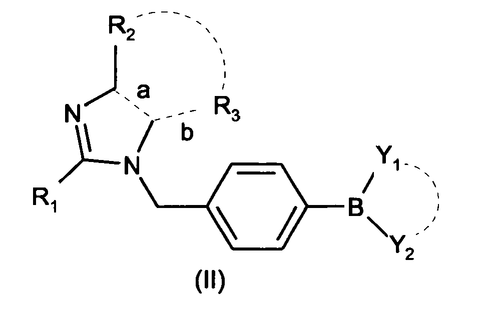 with a compound of formula (VII)

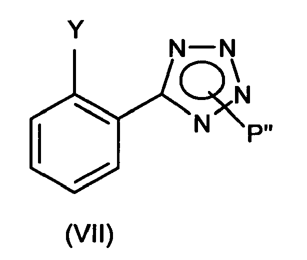

in an appropriate solvent system and in the presence of a metallic compound and a base; where P" is H or a protective group P'; Y is a leaving group such as chlorine, bromine, iodine, methanesulfonyloxy, toluensulfonyloxy, benzenesulfonyloxy or trifluoromethanesulfonyloxy; and each of R₁, R₂ and R₃ is a group as defined above, or an intermediate or protected form thereof which can be transformed to such a group, and thereafter as necessary transforming said intermediate or protected forms of the R₁, R₂ and R₃ groups to R₁, R₂ and R₃ groups as defined above; and optionally when P" is a protective group P', submitting the compound obtained to a deprotection reaction; and optionally the process includes an additional step of converting the reaction product to salt form.

### DETAILED DESCRIPTION OF THE INVENTION

Preferred compounds of formula (II) are those where R₁ is butyl, R₂ forms a spiro-fused ring of 5 carbon atoms with the carbon atom of the heterocyclic ring, a is a single bond, b is a double bond and R₃ is O.

Also preferred compounds of formula (II) are those where R₁ is butyl, R₂ is Cl, a is a double bond, b is a single bond and R₃ is a hydroxymethyl or a formyl radical.

Also preferred compounds of formula (II) are those where R₁ is a (C₁-C₄)-alkoxy radical; a is a double bond; and R₂ and R₃ are bound together forming with the heterocyclic ring a fused benzene ring substituted by a radical selected from (C₁-C₆)-alkoxycarbonyl and
1-{[(cyclohexyloxy)carbonyl]oxy}ethoxycarbonyl. More preferred compounds are those where R₁ is an ethoxy radical, and R₂ and R₃ are bound together forming with the heterocyclic ring a fused benzene ring substituted by an ethoxycarbonyl radical or a methoxycarbonyl radical.

In a preferred embodiment, compounds of formula (II) are those where Y₁ and Y₂ are each independently selected from the group consisting of: hydroxy, methoxy, ethoxy and phenoxy, or alternatively, Y₁ and Y₂ together with the boron atom form a cyclic structure, wherein Z is (CH₂)ᵣCRᵤRᵥ(CH₂)ₛ or CRᵤRᵥ(CH₂)ₜCRᵤRᵥ; r and s are integers from 0 to 4 with the condition that r and s are not both 0; t is 0, and Rᵤ and Rᵥ are each independently selected from methyl and phenyl. In a more preferred embodiment, compounds of formula (II) are those where Y₁ and Y₂ are hydroxy. In other preferred embodiment compounds of formula (II) are those where Y₁ and Y₂ together with the boron atom form a cyclic structure, where Z is CH₂C(CH₃)₂CH₂. In another preferred embodiment compounds of formula (II) are those where Y₁ and Y₂ together with the boron atom form a cyclic structure, wherein Z is C(CH₃)₂C(CH₃)₂.

The most preferred compounds of formula (II) are those of the following list:
4-(2-n-butyl-4-chloro-5-formylimidazol-1-ylmethyl)benzeneboronic acid;
2-n-butyl-4-chloro-1-[4-(5,5-dimethyl-[1,3,2]dioxaborinan-2-yl)benzyl]-1H-imidazole-5-carbaldehyde;
4-(2-n-butyl-4-chloro-5-hydroxymethylimidazol-1-ylmethyl)benzeneboronic acid;
2-n-butyl-4-chloro-1-[4-(5,5-dimethyl-[1,3,2]dioxaborinan-2-yl)benzyl]-1 H-imidazole-5-methanol;
4-(2-n-butyl-4-spirocyclopentane-2-imidazolin-5-one-1-ylmethyl)benzeneboronic acid;
2-n-butyl-3-[4-(5,5-dimethyl-[1,3,2]dioxaborinan-2-yl)benzyl]-1,3-diazaspiro[4.4]non-1-ene-4-one;
ethyl 1-[(5,5-dimethyl-[1,3,2]dioxaborinan-2-yl)methyl]-2-ethoxybenzimidazole-7-carboxylate;
methyl 1-[(5,5-dimethyl-[1,3,2]dioxaborinan-2-yl)methyl]-2-ethoxybenzimidazole-7-carboxylate; and
2-n-butyl-4-chloro-1 -[4-(4,4,5,5-tetramethyl-[1,3,2]dioxoborolan-2-yl)benzyl]-1H-imidazole-5-carbaldehyde.

As previously described, compounds of formula (II) are prepared reacting a compound of formula (III) with a boron compound of formula (IV).

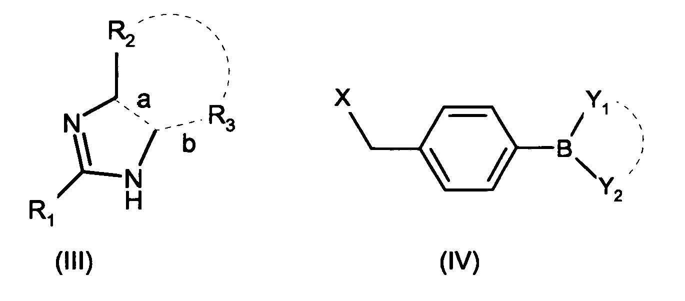

Preferably, the leaving group X is bromine. The reaction is carried out in the presence of a base and a solvent appropriate to the reagents and materials employed and suitable for the transformation being effected. Preferably, the base is an inorganic base selected from alkaline metal hydride such as sodium hydride, alkaline metal hydroxide such as sodium or potassium hydroxide, alkaline metal carbonate such as sodium or potassium carbonate and metal alkoxide such as sodium methoxide. More preferably, the base is potassium carbonate. Preferably the solvent is an aprotic polar solvent such as dimethylformamide.

Also as previously described, compounds of formula (II) wherein R₁ is (C₁-C₄)-alkoxy, a is a double bond, and R₂ and R₃ are bound together forming with the heterocyclic ring a fused benzene ring substituted by a radical selected from the carboxyl, (C₁-C₆)-alkoxycarbonyl and
1-{[(cyclohexyloxy)carbonyl]oxy}ethoxycarbonyl, can also be prepared by a process comprising the steps of:
(a) reacting a compound of formula (V) 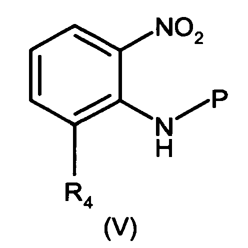 with the boron compound of formula (IV) defined above in the presence of a base and an appropriate solvent; followed by the cleavage of the protective amino group P and the reduction of the nitro group to yield a compound of formula (VI); and 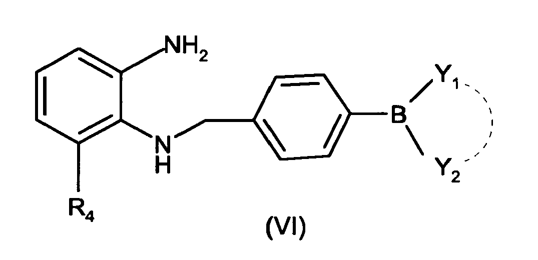
(b) treating the compound (VI) with alkyl orthocarbonate or a carbonylating agent, to give a compound of formula (II');

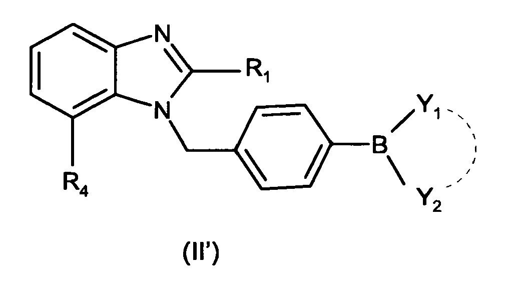

A preferred compound obtained by the previous process is the ethyl 1-[(5,5-dimethyl-[1,3,2]dioxaborinan-2-yl)methyl]-2-ethoxybenzimidazole-7-carboxylate. Another preferred compound obtained by this process is the methyl 1-[(5,5-dimethyl-[1,3,2]dioxaborinan-2-yl)methyl]-2-ethoxybenzimidazole-7-carboxylate.

Preferably, the base is an alkaline metal hydride, such as sodium hydride or an alkaline metal carbonate such as potassium carbonate. Also preferably, the solvent is an aprotic polar solvent or an ether. More preferably, the solvent is dimethylformamide.

A variety of protective groups P may be used for the protection of the amino group of compound (V). Preferred amino protective groups P are carbamates, amides, sulfonamides, an allyl, a benzyl and a mono or a di-substituted benzyl. Examples of protective groups include t-butoxycarbonyl (BOC), 9-fluorenylmethoxycarbonyl (Fmoc), benzyloxycarbonyl (Cbz), allyl, 4-methoxybenzyl, 2,4-dimethoxybenzyl and *N*-benzylamine. The most preferred one is t-butoxycarbonyl (BOC). The conditions for carrying out the deprotection reaction depend on the protective group used. For instance, when a t-butoxycarbonyl (BOC) is used, the deprotection reaction can be carried out with a mixture of trifluoroacetic acid and methylene chloride.

Likewise, the reduction of the nitro group to an amino group can also be carried out with several reducing agents (e.g. tin chloride) or by catalytic hydrogenation.

Each of the R groups and Y groups of the compounds of formula (III), (IV), (V), (VI) and (II') are groups as defined above for the compound of formula (II), or an intermediate or protected form thereof which can be transformed to such a group. When it is necessary the processes include the transformation of said intermediate or protected forms of the R and Y groups to R groups and Y groups as defined for the compound of formula (11). For example a compound with R₃ = formyl group may be easily converted to the corresponding compound with a hydroxymethyl group by a variety of reducing conditions. An example of a suitable reducing agent for effecting this transformation is sodium borohydride. Cyclic boron compounds of the present invention can be obtained from the corresponding boronic acid compounds by reaction with an appropriate substituted alkanediol. For instance, Example 2 illustrates the preparation of 2-n-butyl-4-chloro-1-[4-(5,5-dimethyl-[1,3,2]dioxaborinan-2-yl)benzyl]-1H-imidazole-5-carbaldehyde by reaction of 4-(2-n-butyl-4-chloro-5-formylimidazol-1-ylmethyl)benzeneboronic acid with 2,2-dimethyl-1,3-propanediol.

Compounds of formula (VI) are also part of the present invention. A preferred compound of formula (VI) is ethyl 3-amino-2-{[(5,5-dimethyl-[1,3,2]dioxaborinan-2-yl)methyl]amino}benzoate.

Compounds of formula (II) are useful intermediates for the preparation of compounds of formula (I), or a pharmaceutical salt thereof;

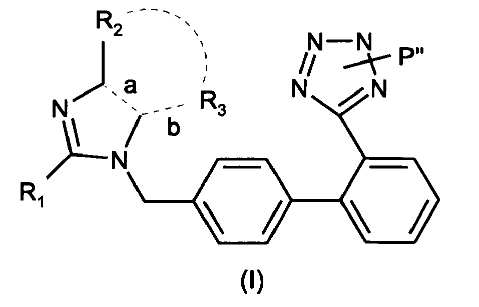

In the previous formula a and b are single or double bond, P" is H or a protective group P'; R₁ is a radical selected from (C₁-C₄)-alkyl and (C₁-C₄)-alkoxy; R₂ is Cl; or alternatively R₂ forms a spiro-fused ring from 4 to 6 carbon atoms with the carbon atom of the heterocyclic ring; R₃ is O or a radical selected from the group consisting of hydroxymethyl and formyl; or alternatively, R₂ and R₃ are bound together forming with the heterocyclic ring a fused benzene ring substituted by a radical selected from the group consisting of carboxyl, (C₁-C₆)-alkoxycarbonyl and
1-{[(cyclohexyloxy)carbonyl]oxy}-ethoxycarbonyl; with the condition that: (i) when a is a single bond, b is a double bond, R₂ forms a spiro-fused ring from 4 to 6 carbon atoms with the carbon atom of the heterocyclic ring and R₃ is O; and (ii) when a is a double bond, b is a single bond, R₂ is Cl; and R₃ is a radical selected from the group consisting of hydroxymethyl and formyl, which are optionally protected; or alternatively, R₂ and R₃ are bound together forming with the heterocyclic ring a fused benzene ring, substituted by a radical selected from the group consisting of carboxyl, (C₁-C₆)-alkoxycarbonyl and 1-{[(cyclohexyloxy)carbonyl]oxy}ethoxycarbonyl; The preparation process comprises a coupling reaction of a compound of formula (II), as previously defined, 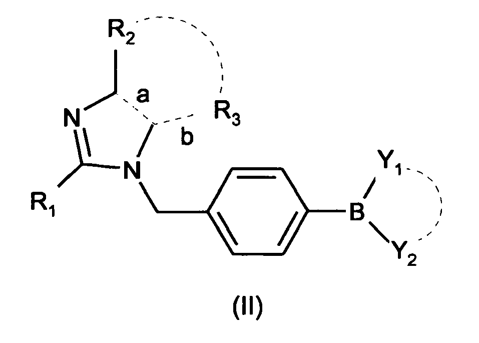 with a compound of formula (VII) 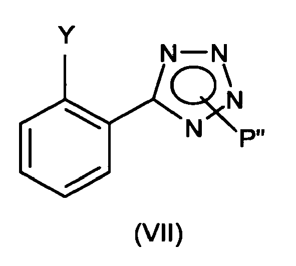 wherein P" is H or a protective group P'; Y is a group such as chlorine, bromine, iodine, methanesulfonyloxy, toluensulfonyloxy, benzenesulfonyloxy or trifluoromethanesulfonyloxy, preferably bromine; and each of R₁, R₂ and R₃ is a group as defined above, or an intermediate or protected form thereof which can be transformed to such a group, and thereafter as necessary transforming said intermediate or protected forms of the R₁, R₂ and R₃ groups to R₁, R₂ and R₃ groups as defined above; and optionally the process includes an additional step of converting the reaction product to salt form.

The reaction of the new substituted heterocyclic methylphenyl boronic compounds of formula (II) with the (halophenyl)tetrazole compound of formula (VII) is known in the art as Suzuki coupling reaction. It is carried out in an appropriate solvent system and in the presence of a metallic compound.

In a preferred embodiment of this preparation process, compound of formula (I) is irbesartan (R₁ is butyl, R₂ forms a spiro-fused ring of 5 carbon atoms with the carbon atom of the heterocyclic ring, a is a single bond, b is a double bond, R₃ is O and P" is H).

In another preferred embodiment of this preparation process, compound of formula (I) is losartan (R₁ is butyl, R₂ is Cl, a is a double bond, b is a single bond, R₃ is a hydroxymethyl and P" is H).

In another preferred embodiment of this preparation process, compound of formula (I) is a precursor of losartan (R₁ is butyl, R₂ is Cl, a is a double bond, b is a single bond, R₃ is formyl and P" is trityl).

In another preferred embodiment, R₁ is a (C₁-C₄)-alkoxy radical; a is a double bond; R₂ and R₃ are bound together forming with the heterocyclic ring a fused benzene ring substituted by a radical selected from the group consisting of carboxyl, (C₁-C₆)-alkoxycarbonyl and 1-{[(cyclohexyloxy)carbonyl]oxy}ethoxycarbonyl and P" is H or triphenylmethyl (trityl). In a specially preferred embodiment compound of formula (I) is candesartan (R₁ is a ethoxy radical, and R₂ and R₃ are bound together forming with the heterocyclic ring a fused benzene ring substituted by a carboxyl, P" is H). In another specially preferred embodiment compound of formula (I) is candesartan cilexetil (R₁ is a ethoxy radical, and R₂ and R₃ are bound together forming with the heterocyclic ring a fused benzene ring substituted by a (1-{[(cyclohexyloxy)carbonyl]oxy}-ethoxycarbonyl radical, P" is H). In an also specially preferred embodiments, R₁ is a ethoxy radical, R₂ and R₃ are bound together forming with the heterocyclic ring a fused benzene ring substituted by a ethoxycarbonyl radical, and P" is trityl, or R₁ is a ethoxy radical, R₂ and R₃ are bound together forming with the heterocyclic ring a fused benzene ring substituted by a methoxycarbonyl radical, and P" is trityl.

Preferably the metallic compound is selected from palladium, nickel, a metallic salt and a metallic complex. More preferably, the metallic compound is a Pd complex added or formed in situ. Examples of suitable metallic compounds include tetrakis(triphenylphosphine)palladium (0), (Pd(PPh₃)₄); dichloro[1,1'-bis(diphenylphosphino)ferrocene]palladium (II), (PdCl₂(dppf)); 1,4-bis(diphenylphosphino)butane palladium (II) chloride, (PdCl₂(dppb)); palladium black; dichlorobis(tricyclohexylphosphine) palladium (II), (PdCl₂(PCy₃)₂); dichloro[1,1'-bis(di-tert-butylphosphino)ferrocene]palladium (II), (PdCl₂(dtbp)); palladium black; palladium (II) chloride; palladium (II) acetate; mixtures of the previously mentioned catalyst with phosphines, preferably with triphenylphosphine and palladium catalysts over polymeric supports. The most preferably metallic compounds are Pd(PPh₃)₄, PdCl₂(dppf), and Pd(OAc)₂/PPh₃.

The best conditions to carry out the process vary according to the parameters considered by the person skilled in the art, such as the starting materials, temperature and similar. Such reaction conditions may be easily determined by the person skilled in the art by routine tests, and with the teaching of the examples included in this document. Likewise the selection of the metallic compound to be used will be determined by the person skilled in the art by routine tests. By way of example suitable metallic compounds to carry out the transformation of 2-n-butyl-4-chloro-1-[4-(5,5-dimethyl-[1,3,2]dioxaborinan-2-yl)benzyl]-1H-imidazole-5-carbaldehyde to losartan are PdCl₂(dppf), (Ph₃P)₄Pd, and palladium catalyst over a polymeric support. Suitable metallic compounds to carry out the transformation of 4-(2-n-butyl-4-chloro-5-formylimidazol-1-ylmethyl)benzeneboronic acid to losartan are Pd(OAc)₂/PPh₃, Pd(PPh₃)₄ and PdCl₂(dppf); and a suitable metallic compound to carry out the transformation of 2-n-butyl-4-chloro-1-[4-(5,5-dimethyl-[1,3,2]dioxoborinan-2-yl)benzyl-1 H-imidazole-5-methanol is PdCl₂(dppf).

Preferably, the solvent system is selected from water, an organic solvent and mixtures of water and one or more organic solvents. More preferably, the solvent is selected from aliphatic or aromatic (C₆-C₈) hydrocarbons such as toluene, xylene; an aprotic polar solvent such as dimethylformamide; aliphatic ethers such as dimethoxyethane, diethoxymethane, diglyme, dioxane, tetrahydrofuran and ethyl ether, and aliphatic (C₁-C₅) alcohols such as ethyl, propyl, isopropyl, butyl, isobutyl, tert-butyl, pentyl, isopentyl and tert-pentyl alcohol. Even more preferably, the solvent is tetrahydrofuran or a mixture of dimethylformide/toluene/water.

There are a variety of bases that can be used to carry out the reaction. Preferably, the base is selected from alkaline metal carbonate, alkaline metal phosphate, a tertiary amine, alkaline metal alkoxide, alkaline metal acetate and alkaline metal fluoride. More preferably, the tertiary amine is triethylamine. Also more preferably the base is selected from potassium carbonate and potassium phosphate.

Preferably, the reaction is carried out at a temperature between ambient temperature and the reflux of the solvent used. More preferably, the reaction temperature is between 66-85 °C.

In a preferred embodiment, P" is a protective group. The protective group of the tetrazole moiety can be introduced and removed by procedures known in the art (cf. Protective Groups in Organic Synthesis, Wiley-Interscience, (1999)). The specific reaction conditions depend on the protective group used. Thus, in embodiments in which the tetrazole group is protected, the process include a further step in which the protective group is cleaved from the tetrazole ring. Preferably, the protective group is the trityl, but other protective groups can be used for purposes of the present invention. For instance, when trityl group is used as the protective group of the tetrazole moiety, it can be deprotected either in acidic or basic conditions. Preferably, the deprotection is carried out in acidic conditions, for example, HCl in a suitable solvent such as methanol or a mixture of dioxane/water.

Likewise, when it is necessary the process includes the transformation of intermediates or protected forms of the R groups to R groups as defined above for the compound of formula (I). For example compounds wherein R₃ is a formyl group may be easily converted to the corresponding compound with a hydroxymethyl group by a variety of reducing conditions. Also as a way of example, compounds of formula (I), wherein R₁ is a (C₁-C₄)-alkoxy, a is a double bond, and R₂ and R₃ are bound together forming with the heterocyclic ring a fused benzene ring substituted by a radical selected from the group consisting of carboxyl, (C₁-C₆)-alkoxycarbonyl and
1-{[(cyclohexyloxy)carbonyl]oxy}ethoxycarbonyl, prepared by the preceeding process, may be transformed into a compound of formula (I) by a nucleofilic reaction, acylation or esterification and/or deprotection. Thus, candesartan can be obtained from the corresponding precursor with an ester moiety prepared by the process previously described, and followed by saponification. Preferably, the saponification is carried out with an alkaline metal hydroxide, and more preferably with lithium hydroxide. If the protective group of the tetrazole is cleaved with the saponification conditions used, preferably the candesartan is protected again before the preparation of candesartan cilexetil. Such compound can be obtained from candesartan by procedures known in the art (cf. EP 459.136). Thus, candesartan cilexetil is obtained from candesartan with the tetrazole moiety N-protected with a suitable protective group such as trityl, previously obtained by the process of the present invention, by reaction with 1-(cyclohexyloxycarbonyloxy)ethyl iodide in the presence of a base and a suitable solvent and followed by deprotection of the tetrazole moiety.

Finally, compounds of formula (I) obtained by the process of the present invention may be converted into pharmaceutically acceptable salts, and salts may be converted into free compounds, by conventional methods. For instance, losartan can be converted in its potassium salt by standard procedures described in the art.

An advantage of the present invention lies in the fact that this preparation process allows to prepare all compounds of the present invention from a common intermediate compound, a N-protected- 5-(2-halogenphenyl)-1 H-tetrazole,for instance, 5-(2-bromophenyl)- 1-triphenylmethyl-1 H-tetrazole. This intermediate is easily obtained from commercial starting materials. Furthermore, it is advantageous because the tetrazole moiety, which easily decomposed in the reaction media, is introduced in the last step. Moreover, the process of the present invention is particularly advantageous in its practical industrial realization because it avoids the use of azide derivatives which are reactants difficult to handle and also it avoids the use of expensive biphenyl intermediates.

Throughout the description and claims the word "comprise" and variations of the word, are not intended to exclude other technical features, additives, components, or steps. The abstract of this application is incorporated herein as reference. Additional objects, advantages and features of the invention will become apparent to those skilled in the art upon examination of the description or may be learned by practice of the invention. The following examples are provided by way of illustration, and they are not intended to be limiting of the present invention.

### EXAMPLES

### Example 1: Preparation of 4-(2-n-butyl-4-chloro-5-formylimidazol-1-ylmethyl) benzeneboronic acid

A solution of 4-bromomethylbenzeneboronic acid (3.63 g) in anhydrous dimethylformamide (9.8 mL) was slowly added (2 h) to a mixture of 2-*n*-butyl-4-chloro-1H-imidazole-5-carbaldehyde (2 g) and K₂CO₃ (5.92 g) finely pulverized in anhydrous dimethylformamide (8.0 mL) under inert atmosphere. When the addition was completed, 4-bromomethylbenzeneboronic acid (0.48 g) was further added. The reaction was stirred for 1 h. The mixture was filtered and the solid was washed with ethyl acetate (48 mL). The filtrate and washings were collected and poured on to H₂O (60 mL). The aqueous layer was adjusted to neutral pH with 1 M HCI. The organic layer was washed with aqueous NaCl saturated solution (brine, 96 mL x 4) and dried over anhydrous Na₂SO₄, then filtered and evaporated to dryness. The residue was stirred in a mixture of dichloromethane (34.3 mL) and 1 M HCl (34.3 mL) at 0-5 °C for 2 h. The solid was filtered, washed with cold H₂O (7 mL) and dried at room temperature giving 2.93 g (86 %). ¹H NMR (400 MHz, CDCl₃) δ 9.73 (s, 1 H, CHO), 7.70 (d, *J* = 7.6 Hz, 2 H, H-Ar), 7.01 (d, *J* = 7.6 Hz, 2 H, H-Ar), 5.55 (s, 2 H, CH₂), 2.61 (t, *J* = 7.8 Hz, 2 H, CH₂), 1.64 (m, 2 H, CH₂), 1.32 (m, 2 H, CH₂), 0.87 (t, *J* = 7.0 Hz, 3 H, CH₃) ppm. ¹³C NMR (100 MHz, CDCl₃) δ 177.9 (CHO), 154.7 (C-*imidazole*)*,* 142.8 (C-*ipso*-Ar-B), 137.2 (C), 134.1 (CH), 128.5 (C), 125.3 (CH), 124.1 (C), 48.1 (CH₂), 28.9 (CH₂), 26.2 (CH₂), 20.1 (CH₂), 13.3 (CH₃) ppm. IR (υ): 3402 (OH), 1673 (C=O), 1338 (B-O and C-O), 1193 (B-C) cm⁻¹. MS-ES(+): 321 (M⁺+1)

### Example 2: Preparation of 5-(2-bromophenyl)-1-triphenylmethyl-1H-tetrazole

Et₃N (2.75 mL) was added to a solution of 5-(2-bromophenyl)-1H-tetrazole (4 g) in anhydrous tetrahydrofuran (32 mL). The mixture was heated to 40 °C under inert atmosphere. A solution of triphenylmethyl chloride (5.56 g) in anhydrous tetrahydrofuran (11.1 mL) was added dropwise. The reaction was stirred at 40 °C for 1 h. The reaction mixture was cooled to 0-5 °C and filtered. The solid was washed with cold tetrahydrofuran (1.6 mL). The filtrate and washings were combined and evaporated to dryness. The residue was stirred in a mixture acetone: H₂O (48 mL, 1:1) for 1 h. The solid was filtered, washed with cold water (12 mL) and dried at room temperature giving (8.17 g, quantitative yield). ¹H NMR (400 MHz, CDCl₃) δ 7.88 (dd, J = 7.6 and 1.6 Hz, 1 H, H-Ar), 7.70 (dd, J = 8.4 and 1.2 Hz, 1 H, H-Ar), 7.69-7.17 (m, 17 H, H-Ar) ppm

### Example 3: Preparation of 2-n-butyl-4-chloro-1-[4-(5,5-dimethyl-[1,3,2]dioxaborinan-2-yl)benzyl]-1H-imidazole-5-carbaldehyde

2,2-Dimethyl-1,3-propanediol (547 mg) was added to a solution of 4-(2-n-butyl-4-chloro-5-formylimidazol-1-ylmethyl)benzeneboronic acid (1.5 g) in dry tetrahydrofuran (15 mL). The reaction was stirred at room temperature overnight. The solvent was evaporated to dryness. The residue was treated with dichloromethane (44 mL) and washed with H₂O (29 mL x 3). The organic layer was dried over anhydrous Na₂SO₄ and filtered. The solvent was evaporated to dryness yielding 1.69 g (63%). The product was used in the next reaction step without further purification. ¹H NMR (400 MHz, CDCl₃) δ 9.75 (s, 1 H, CHO), 7.74 (d, *J* = 8.4 Hz, 2 H, H-Ar), 7.01 (d, *J* = 8 Hz, 2 H, H-Ar), 5.56 (s, 2 H, CH₂), 3.75 (s, 4 H, CH₂O), 2.59 (t, *J* = 7.8 Hz, 2 H, CH₂), 1.65 (m, 2 H, CH₂), 1.32 (m, 2 H, CH₂), 1.01 (s, 6 H, CH₃), 0.86 (t, *J =* 7.4 Hz, 3 H, CH₃) ppm. ¹³C-NMR (100 MHz, CDCl₃) δ 13.8 (CH₃), 22.1 (CH₃), 22.6 (CH₂), 26.7 (CH₂), 29.4 (CH₂), 32.1 (C), 48.5 (CH₂), 72.5 (CH₂), 125.7 and 134.7 (CH-Ar), 124.6, 134.5 and 138.1 (C-*ipso*-Ar), 143.2 (C-*ipso*-Ar-B), 154.9 (C-*imidazole*), 178.1 (CHO) ppm. IR (υ): 1669 (C=O), 1317 (B-O and C-O), 1132 (B-C) cm⁻¹. MS-ES(+): 389 (M⁺+1)

### Example 4: Preparation of 2-n-butyl-4-chloro-1-[4-(4,4,5,5-tetramethyl-[1,3,2]dioxaborolan-2-yl)benzyl]-1H-imidazole-5-carbaldehyde

2,3-Dimethyl-2,3-butanediol (163 mg) was added to a solution of 4-(2-n-butyl-4-chloro-5-formylimidazol-1-ylmethyl)benzeneboronic acid (400 mg) in dry tetrahydrofuran (4 mL). The reaction was stirred at room temperature overnight. The solvent was evaporated to dryness. The residue was treated with dichloromethane (12 mL) and washed with H₂O (8 mL x 3). The organic layer was dried over anhydrous Na₂SO and filtered. The solvent was evaporated to dryness yielding 414 mg. The product was used in the next reaction step without further purification. ¹H NMR (400 MHz, CDCl₃) δ 9.74 (s, 1 H, CHO), 7.76 (d, *J* = 8 Hz, 2 H, H-Ar), 7.02 (d, *J* = 8 Hz, 2 H, H-Ar), 5.57 (s, 2 H, CH₂), 2.59 (t, *J* = 7.8 Hz, 2 H, CH₂), 1.66 (m, 2 H, CH₂), 1.36-1.32 (m, 14 H, CH₂ and 4 CH₃), 0.87 (t, *J* = 7.4 Hz, 3 H, CH₃) ppm. ¹³C NMR (100 MHz, CDCl₃) 13.6 (CH₃), 22.4 (CH₂), 24.8 (CH₃), 26.5 (CH₂), 29.1 (CH₂), 48.3 (CH₂), 83.9 (C), 125.5 and 135.4 (CH-Ar), 124.3, 135.2 and 138.5 (C-*ipso*-Ar), 143 (C-*ipso*-Ar-B), 154.6 (C-*imidazole*), 177.9 (CHO). IR (υ): 1668 (C=O), 1361 (B-O and C-O), 1144 (B-C) cm⁻¹.

### Example 5: Preparation of 4-(2-n-butyl-4-chloro-5-hydroxymethylimidazol-l-ylmethyl)benzeneboronic acid

NaBH₄ (61 mg) was added to a solution of 4-(2-n-butyl-4-chloro-5-formylimidazol-1-ylmethyl)benzeneboronic acid (500 mg) in MeOH (2.5 mL) at 0-5 °C under inert atmosphere. The reaction was stirred at room temperature for 1 h. H₂O (5 mL) was added dropwise and the mixture was extracted with ethyl acetate (25 mL x 2). The organic layers were combined and dried over anhydrous Na₂SO₄ and filtered. The solvent was evaporated to dryness yielding 454 mg (91%). ¹H NMR (400 MHz, CDCl₃) δ 7.72 and 7.58 (2 d, *J* = 7.4 Hz, 2 H, H-Ar), 6.98 (m, 2 H, H-Ar), 5.22 (s, 2 H, CH₂), 4.43 (s, 2 H, CH₂OH), 3.11 (s, 2 H, OH), 2.53 (t, *J* = 7.6 Hz, 2 H, CH₂), 1.58 (m, 2 H, CH₂), 1.29 (m, 2 H, CH₂), 0.85 (t, *J* = 7.4 Hz, 3 H, CH₃) ppm. ¹³C-NMR (100 MHz, CDCl₃) δ 148.3 (C), 137.8 (C), 134.2 (CH-Ar), 133.8 (C), 126.4 (C), 125.1 (C), 124.8 (CH-Ar), 52.2 (CH₂), 47.3 (CH₂), 29.5 (CH₂), 26.4 (CH₂), 22.1 (CH₂), 13.3 (CH₃) ppm. IR (υ): 3737 (OH), 1315 (B-O and C-O), 1099 (B-C) cm⁻¹. MS-ES(+): 323 (M⁺+1)

### Example 6 : Preparation of 2-n-butyl-4-chloro-1-[4-(5,5-dimethyl-[1,3,2]dioxaborinan-2-yl)benzyl]-1H-imidazole-5-methanol

NaBH₄ (61 mg) was added to a solution of 2-n-butyl-4-chloro-1-[4-(5,5-dimethyl-[1,3,2]dioxaborinan-2-yl)benzyl]-1*H*-imidazole-5-carbaldehyde (384 mg) in MeOH (1.9 mL) at 0-5 °C under inert atmosphere. The reaction was stirred at 0-5 °C for 1 h. H₂O (4 mL) was added dropwise and the mixture was extracted with ethyl acetate (20 mL x 2). The organic layers were combined and dried over anhydrous Na₂SO₄ and filtered. The solvent was evaporated to dryness giving 396 mg (quantitative yield). ¹H NMR (400 MHz, CDCl₃) δ 7.74 (d, *J* = 8 Hz, 2 H, H-Ar), 6.96 (d, *J* = 8 Hz, 2 H, H-Ar), 5.22 (s, 2 H, CH₂), 4.45 (s, 2 H, CH₂-OH), 3.75 (s, 4 H, CH₂O), 2.52 (t, *J* = 8 Hz, 2 H, CH₂), 1.61 (m, 2 H, CH₂), 1.29 (m, 2 H, CH₂), 1.01 (s, 6 H, CH₃), 0.84 (t, *J* = 7.4 Hz, 3 H, CH₃) ppm. ¹³C-NMR (100 MHz, CDCl₃) δ 13.5 (CH₃), 21.7 (CH₃), 22.2 (CH₂), 26.6 (CH₂), 29.6 (CH₂), 31.8 (C), 47.5 (CH₂), 52.6 (CH₂), 72.3 (CH₂), 124.9 and 134.5 (CH-Ar), 125, 133.9 and 138.3 (C-*ipso*-Ar), 143.5 (C-*ipso*-Ar-B), 148.6 (C*-imidazole*) ppm. IR (υ): 3236 (OH), 1317 (B-O and C-O), 1133 (B-C) cm⁻¹. MS-ES(+): 391 (M⁺+1)

### Example 7 : Preparation of 2-n-butyl-4-chloro-1-[{2'-[1-(triphenylmethyl)-1H-tetrazol-5-yl]-1,1'-biphenyl-4-yl}methyl]-1H-imidazole-5-carbaldehyde

A Schlenk tube was charged with 5-(2-bromophenyl)-1-triphenylmethyl-1*H-*tetrazole (100 mg), anhydrous K₂CO₃ (72 mg), tetrahydrofuran (0.2 mL), diethoxymethane (0.7 mL) and H₂O (9 µL). The suspension was purged and degassed. A portion of tetrakis(triphenylphosphine)palladium (0) (Pd(PPh₃)₄) and 4-(2-*n*-butyl-4-chloro-5-formylimidazol-1-ylmethyl)benzeneboronic acid were added, the mixture was purged and degassed, and refluxed for 1 h. Then, another portion of Pd(PPh₃)₄ and 4-(2-*n*-butyl-4-chloro-5-formylimidazol-1-ylmethyl)benzeneboronic acid were added, the mixture was purged and degassed and refluxed for further 1 h. The process was completed after 2 more additions of the catalyst and the boronic acid. The reaction was heated at reflux overnight. After cooling to room temperature, the reaction mixture was filtered and the cake was washed with ethyl acetate (7 mL). The filtrate was washed with H₂O (3 mL) and the aqueous layer was extracted with ethyl acetate (5 mL). The organic layer was dried over anhydrous Na₂SO₄, filtered and evaporated to dryness. The residue was purified by column chromatography (eluant: cyclohexane/ethyl acetate, 9:1) to yield 41 mg (29%).

### Example 8: Preparation of 2-n-butyl-4-chloro-1-[{2'-[1-(triphenylmethyl)-1H-tetrazol-5-yl]-1,1'-biphenyl-4-yl}methyl]-1H-imidazole-5-carbaldehyde

A Schlenk tube was charged with 2-n-butyl-4-chloro-1-[4-(5,5-dimethyl-[1,3,2]dioxaborinan-2-yl)benzyl)-1*H*-imidazole-5-carbaldehyde (309 mg), 5-(2-bromophenyl)-1-triphenylmethyl-1 H-tetrazole (250 mg), anhydrous K₃PO₄ (337 mg) and anhydrous tetrahydrofuran (5.5 mL). The suspension was purged and degassed. Pd(PPh₃)₄ (18 mg) was added, the mixture was purged and degassed. The reaction was refluxed for 48 h. After cooling to room temperature, the reaction mixture was filtered, the cake was washed with ethyl acetate. The filtrate and washings were combined and evaporated to dryness. The residue was purified by column chromatography (eluant: cyclohexane/ethyl acetate, 9:1) to yield 81 mg (23%).

### Example 9 : Preparation of 2-n-butyl-4-chloro-1-[{2'-[1-(triphenylmethyl)-1H-tetrazol-5-yl]-1,1'-biphenyl-4-yl}methyl]-1H-imidazole-5-carbaldehyde

A Schlenk tube was charged with 2-n-butyl-4-chloro-1-[4-(5,5-dimethyl-[1,3,2]dioxaborinan-2-yl)benzyl)-1*H*-imidazole-5-carbaldehyde (190 mg), 5-(2-bromophenyl)-1-triphenylmethyl-1*H*-tetrazole (229 mg), anhydrous K₃PO₄ (317 mg) and anhydrous tetrahydrofuran (5 mL). The suspension was purged and degassed. Dichloro[1,1-bis(diphenylphosphino)ferrocene]palladium (II) (PdCl₂(dppf)) (13 mg) was added, the mixture was purged and degassed. The reaction was refluxed for 72 h. After cooling to room temperature, the reaction mixture was filtered, the cake was washed with dichloromethane. The filtrate and washings were combined and evaporated to dryness. The residue was purified by column chromatography (eluant: cyclohexane/ethyl acetate, 9:1) to yield 212 mg (65%).

### Example 10: Preparation of 2-n-butyl-4-chloro-1-[{2'-[1-(triphenylmethyl)-1H-tetrazol-5-yl]-1,1'-biphenyl-4-yl}methyl]-1H-imidazole-5-carbaldehyde

A Schlenk tube was charged with 4-(2-n-butyl-4-chloro-5-formylimidazol-1-ylmethyl)benzeneboronic acid (199 mg), 5-(2-Bromophenyl)-1-triphenylmethyl-1*H*-tetrazole (250 mg), anhydrous K₂CO₃ (366 mg), toluene (3.45 mL), dimethylformamide (0.34 mL) and H₂O (1.04 mL). The suspension was purged and degassed. Dichloro[1,1-bis(diphenylphosphino)ferrocene]-palladium (II) (PdCl₂(dppf)) (13 mg) was added, the mixture was purged and degassed. The reaction was heated at 85 °C for 48 h. After cooling to room temperature, ethyl acetate (3.4 mL) and H₂O (0.6 mL) were added, the mixture was stirred for 10 min. The organic layer was washed with H₂O (3.4 mL), dried over anhydrous Na₂SO₄, filtered and evaporated to dryness. The residue was purified by column chromatography (eluant: cyclohexane/ethyl acetate, 95:5) to yield 130 mg (37%).

### Example 11: Preparation of 2-n-butyl-4-chloro-1-[{2'-[1-(triphenvimethyl)-1H-tetrazol-5-yl]-1,1'-biphenyl-4-yl}methyl]-1H-imidazole-5-carbaldehyde

A Schlenk tube was charged with 2-*n*-butyl-4-chloro-1-[4-(5,5-dimethyl-[1,3,2]dioxaborinan-2-yl)benzyl)-1*H*-imidazole-5-carbaldehyde (190 mg), 5-(2-chlorophenyl)-1-triphenylmethyl-1*H*-tetrazole (207 mg), anhydrous K₃PO₄ (312 mg) and anhydrous tetrahydrofuran (5 mL). The suspension was purged and degassed. Dichloro[1,1-bis(diphenylphosphino)ferrocene]palladium (II) (PdCl₂(dppf)) (12 mg) was added, the mixture was purged and degassed. The reaction was refluxed for 48 h. After cooling to room temperature, the reaction mixture was filtered, the cake was washed with dichloromethane. The filtrate and washings were combined and evaporated to dryness. The residue was purified by column chromatography (eluant: cyclohexane/ethyl acetate, 9:1) to yield 4 mg.

### Example 12: Preparation of 2-n-butyl-4-chloro-1-[{2'-[1-(triphenylmethyl)-1H-tetrazol-5-yl]-1,1'-biphenyl-4-yl}methyl]-1H-imidazole-5-carbaldehyde

A Schlenk tube was charged with 2-*n*-Butyl-4-chloro-1-[4-(4,4,5,5-tetramethyl-[1,3,2]dioxaborolan-2-yl)benzyl]-1*H*-imidazole-5-carbaldehyde (200 mg), 5-(2-bromophenyl)-1-triphenylmethyl-1*H*-tetrazole (229 mg), anhydrous K₃P0₄ (318 mg) and anhydrous tetrahydrofuran (5 mL). The suspension was purged and degassed. Dichloro[1,1-bis(diphenylphosphino)ferrocene]palladium (II) (PdCl₂(dppf)) (13 mg) was added, the mixture was purged and degassed. The reaction was refluxed for 72 h. After cooling to room temperature, the reaction mixture was filtered, the cake was washed with dichloromethane. The filtrate and washings were combined and evaporated to dryness. The residue was purified by column chromatography (eluant: cyclohexane/ethyl acetate, 9:1) to yield 140 mg (43%). ¹H NMR (400 MHz, CDCl₃) δ 0.86 (t, *J =* 7.4 Hz, 3 H, CH₃), 1.29 (m, 2 H, CH₃-C*H*₂-CH₂-), 1.64 (qn, *J =* 7.6 Hz, CH₂-C*H*₂-CH₂-), 2.52 (t, *J* = 7.6 Hz, CH₂-C*H*₂-C-). 5.44 (s, 2 H, Ph-C*H*₂-N-), 6.82-7.94 (m, 23 H, H-Ar), 9.73 (s, 1 H, CHO) ppm

### Example 13: Preparation of 2-n-butyl-4-chloro-1-[{2'-[1-(triphenylmethyl)-1H-tetrazol-5-yl]-1,1'-biphenyl-4-yl}methyl]-1H-imidazole-5-methanol

A Schlenk tube was charged with 2-n-butyl-4-chloro-1-[4-(5,5-dimethyl-[1,3,2]dioxaborinan-2-yl)benzyl)-1*H*-imidazole-5-methanol (145 mg), 5-(2-bromophenyl)-1-triphenylmethyl-1*H* tetrazole (173 mg), anhydrous K₃PO₄ (235 mg) and anhydrous tetrahydrofuran (4 mL). The suspension was purged and degassed. Dichloro[1,1-bis(diphenylphosphino)ferrocene]palladium (II) (PdCl₂(dppf)) (9 mg) was added, the mixture was purged and degassed. The reaction was refluxed for 72 h. After cooling to room temperature, the reaction mixture was filtered, the cake was washed with dichloromethane. The filtrate and washings were evaporated to dryness. The residue was purified by column chromatography (eluant: cyclohexane/ethyl acetate, 9:1) to yield 82 mg (33%).

### Example 14: Preparation of 2-n-butyl-4-chloro-1-[{2'-[1-(triphenylmethyl)-1H tetrazol-5-yl]-1,1'-biphenyl-4-yl}methyl]-1H-imidazole-5-methanol

A Schlenk tube was charged with 4-(2-*n*-Butyl-4-chloro-5-hydroxymethylimidazol-1-ylmethyl)benzeneboronic acid (200 mg), 5-(2-Bromophenyl)-1-triphenylmethyl-1*H*-tetrazole (290 mg), anhydrous K₂CO₃ (428 mg) and toluene (4 mL), dimethylformamide (0.4 mL) and H₂O (1.2 mL). The suspension was purged and degassed. Dichloro [1,1-bis (diphenylphosphino)ferrocene]palladium (II) (PdCl₂(dppf)) (9 mg) was added, the mixture was purged and degassed. The reaction was refluxed for 48 h. After cooling to room temperature, the reaction mixture was filtered, the cake was washed with dichloromethane. The filtrate and washings were evaporated to dryness. The residue was purified by column chromatography (eluant: cyclohexane/ethyl acetate, 9:1) to yield 37 mg.

### Example 15: Preparation of 2-n-butyl-4-chloro-1-[{2'-[1-(triphenylmethyl)-1H-tetrazol-5-yl]-1,1'-biphenyl-4-yl}methyl]-1H-imidazole-5-methanol

NaBH₄ (12 mg) was added to a solution of 2-n-butyl-4-chloro-1-[{2'-[1-(triphenylmethyl)-1*H*-tetrazol-5-yl]-1,1'-biphenyl-4-yl}methyl]-1*H*-imidazole-5-carbaldehyde (210 mg) in methanol (1 mL) and toluene (0.44 mL) at 0-5 °C under inert atmosphere. The reaction was stirred at room temperature for 1 h. H₂O (2 mL) was added and the mixture was extracted with ethyl acetate (2.4 mL x 2). The organic layers were combined and washed with H₂O (1.2 mL), dried over anhydrous Na₂SO₄, filtered and evaporated to dryness giving 210 mg (94%).The product was used in the next reaction step without further purification. ¹H NMR (400 MHz, CDCl₃) δ 0.86 (t, *J* = 7.4 Hz, 3 H, CH₃), 1.28 (m, 2 H, CH₃-C*H₂*-CH₂-), 1.60 (bs, 1 H, OH), 1.65 (qn, *J* = 8 Hz, CH₂-*CH₂*-CH₂-), 2.49 (t, *J* = 7.8 Hz, CH₂-*CH₂*-C-), 4.29 (s, 2 H, *CH₂*-OH), 5.09 (s, 2 H, *CH₂-*N), 6.75-7.96 (m, 23 H, H-Ar) ppm.

### Example 16: Preparation of 2-n-butyl-4-chloro-1-{[2'-(1H-tetrazol-5-yl)-1,1'-biphenyl-4-yl]methyl}1H-imidazole-5-methanol

A mixture of 2-*n*-butyl-4-chloro-1-[{2'-[1-(triphenylmethyl)-1*H*-tetrazol-5-yl]-1,1'-biphenyl-4-yl}methyl]-1*H*-imidazole-5-methanol (368 mg) and 1,4-dioxane (4.4 mL), water (0.88 mL) and 4 N HCl (0.41 mL) was stirred at room temperature overnight. Water (186 mL) was added and the product was extracted with ethyl acetate (186 mL x 3). The combined organic layers were dried over anhydrous Na₂SO₄, filtered and the solvent was evaporated under reduced pressure. The residue was purified by column chromatography on silica (eluant: cyclohexane/ethyl acetate from 9:1 to ethyl acetate, and further to ethyl acetate/MeOH 8:2) to obtain 2-*n*-butyl-4-chloro-1-{[2'-(1*H*-tetrazol-5-yl)-1,1'-biphenyl-4-yl]methyl}-1*H*-imidazole-5-methanol (156 mg, 67%). ¹H NMR (400 MHz, CDCl₃) δ 0.78 (t, *J* = 7.4 Hz, 3 H, CH₃), 1.24 (m, 2 H, CH₃-CH₂-CH₂-), 1.48 (qn, *J* = 7.6 Hz, CH₂-CH₂-CH₂-), 2.39 (t, *J* = 7.8 Hz, CH₂-CH₂-C-), 4.43 (s, 2 H, C*H*₂-OH), 5.16 (s, 2 H, *CH₂-N),* 6.87 (d, *J* = 8 Hz, 2 H, H-Ar), 7.04 (d, *J* = 8 Hz, 2 H, H-Ar), 7.40 (d, *J* = 7.6 Hz, 1 H, H-Ar), 7.49 (td, *J* = 7.6 and 1.1 Hz, 1 H, H-Ar), 7.57 (td, *J* = 7.5 and 1.1 Hz, 1 H, H-Ar), 7.85 (d, *J* = 7.6 Hz, 1 H, H-Ar) ppm. ¹³C-NMR (100 MHz, CDCl₃) δ 14 (CH₃), 22.7, 26.9 and 30.2 (CH₂), 47.7 (CH₂-N), 52.8 (CH₂-OH), 126.5, 128.5, 130, 131, 131.1 and 131.6 (CH-Ar), 123.6, 125.5, 127.3, 136, 139.4, 141.4, 148.9 and 155.9 (C-*ipso*-Ar) ppm. IR (υ): 3360 (broad, OH and NH) cm⁻¹. MS-ES (+): 423 (M⁺+1).

### Example 17: Preparation of 2-n-butyl-4-chloro-1-{[2'-(1H-tetrazol-5-yl)-1,1'-biphenyl-4-yl]methyl}-1H-imidazole-5-methanol potassium

A mixture of 2-*n*-butyl-4-chloro-1-{[2'-(1*H*-tetrazol-5-yl)-1,1'-biphenyl-4-yl]methyl}-1*H*-imidazole-5-methanol (1 g) in dry methanol (8.75 mL) was warmed to reflux temperature. Then, potassium hydroxide (99 mg) in dry methanol (1.7 ml) was added and the mixture was refluxed for 4 h. The reaction was cooled to room temperature, treated with charcoal and filtered. The filtrate was concentrated to a volume of 1.5-1.75 mL, and after addition of acetonitrile (4.2 mL) again to a volume of 1.5-1.75 mL. Further acetonitrile (4.2 mL) was added and the solution was concentrated to a volume of 3 mL. The suspension was stirred at 0-5°C overnight and the solid was filtered, washed with cold acetonitrile (3 x 1.5 mL) and dried at 60°C overnight to give 231 mg (33%) of the title compound. ¹H NMR (400 MHz, DMSO-d₆) δ 0.79 (t, *J* = 7.4 Hz, 3 H, CH₃), 1.24 (m, 2 H, CH₃-*CH*₂-CH₂-), 1.46 (qn, *J* = 7.6 Hz, CH₂-*CH₂*-CH₂-), 2.48 (bs, 2 H, CH₂-*CH*₂-C-), 4.29 (s, 2 H, *CH*₂-OH), 5.19 (s, 2 H, *CH₂-*N), 5.30 (bs, 1 H, OH), 6.88 (d, *J* = 8 Hz, 2 H, H-Ar), 7.07 (d, *J* = 8 Hz, 2 H, H-Ar), 7.40 (d, *J* = 7.6 Hz, 1 H, H-Ar), 7.49 (td, *J* = 7.6 and 1.1 Hz, 1 H, H-Ar), 7.57 (td, *J* = 7.5 and 1.1 Hz, 1 H, H-Ar), 7.85 (d, *J* = 7.6 Hz, 1 H, H-Ar) ppm. ¹³C-NMR (100 MHz, DMSO-d₆) δ 14.3 (CH₃), 22.3, 26.5 and 29.8 (CH₂), 47.2 (CH₂-N), 52 (CH₂-OH), 125.9, 127.4, 127.9, 130.1, 130.7 and 131.2 (CH-Ar), 125.9, 126.3, 133.3, 135.3, 140.5, 141.8, 148 and 161.4 (C-*ipso*-Ar) ppm. IR (υ): 3734 (OH), 1259 (C-O) cm⁻¹.

### Example 18 : Preparation of 4-(2-n-butyl-4-spirocyclopentane-2-imidazolin-5-one-1-ylmethyl)benzeneboronic acid

A solution of 4-bromomethylbenzeneboronic acid (3.67 g) in anhydrous dimethylformamide (10 mL) was slowly added (2 h) to a mixture of 2-n-butyl-1,3-diazaspiro[4.4]non-1-ene-4-one hydrochloride (2.5 g) and K₂CO₃ (7.48 g) finely pulverized in anhydrous dimethylformamide (10 mL) under inert atmosphere. The mixture was filtered and the solid was washed with ethyl acetate (49 mL). The filtrate and washings were collected and poured on to H₂O (62 mL). The aqueous layer was adjusted to neutral pH with 1 M HCl and the mixture was stirred at 0-5 °C overnight. The solid was filtered, washed with cold H₂O (7.5 mL) and dried at room temperature giving 1.48 g (42 %). ¹H NMR (400 MHz, CDCl₃) δ 7.79, 7.73, 7.59 and 7.12 (4 d, *J* = 7.4 Hz, 4 H, H-Ar), 4.69 (s, 2 H, CH₂), 3.2 (bs, 2 H, OH), 2.29 (t, *J* = 8 Hz, 2 H, CH₂), 1.98 and 1.81 (2 bs, 8 H, -CH₂-CH₂-), 1.52 (m, 2 H, CH₂), 1.31 (m, 2 H, CH₂), 0.85 (t, *J* = 7.2 Hz, 3 H, CH₃) ppm. ¹³C-NMR (100 MHz, CDCl₃) δ 13.4 (CH₃), 22.0 (CH₂), 25.9 (CH₂), 27.5 (CH₂), 28.5 (CH₂), 37.2 (CH₂), 43.5 (CH₂), 76.3 (C), 125.7 (CH-Ar), 134.2 (CH-Ar), 138.0 (C-ipso-Ar), 162.6 (C), 186.6 (CO) ppm. IR (υ):3358 (BO-H), 1648 (C=O), 1419 (B-O and C-O) cm⁻¹. MS-ES(+): 328 (M⁺+1).

### Example 19: Preparation of 2-n-butyl-3-[4-(5,5-dimethyl-[1,3,2]dioxaborinan-2-yl)benzyl]-1,3-diazaspiro[4.4]non-1-ene-4-one

2,2-Dimethyl-1,3-propanediol (178 mg) was added to a solution of 4-(2-n-butyl-4-spirocyclopentane-2-imidazolin-5-one-1-ylmethyl)benzeneboronic acid (500 mg) in dry tetrahydrofuran (5 mL). The reaction was stirred at room temperature overnight. The solvent was evaporated to dryness. The residue was treated with dichloromethane (14.5 mL) and washed with H₂O (9.6 mL x 3). The organic layer was dried over anhydrous Na₂SO₄ and filtered. The solvent was evaporated to dryness yielding 493 mg (82%). The product was used in the next reaction step without further purification. ¹H NMR (400 MHz, CDCl₃) δ 7.76 (d, *J* = 8.4 Hz, 2 H, H-Ar), 7.13 (d, *J* = 8 Hz, 2 H, H-Ar), 4.68 (s, 2 H, CH₂), 3.76 (s, 4 H, CH₂O), 2.26 (t, *J* = 7.8 Hz, 2 H, CH₂), 1.97 (m, 6 H, CH₂), 1.81 (m, 2 H, CH₂), 1.54 (t, J = 7.6 Hz, 2 H, CH₂), 1.29 (m, 2 H, CH₂), 1.01 (s, 6 H, CH₃), 0.84 (t, *J* = 7.4 Hz, 3 H, CH₃) ppm. ¹³C-NMR (100 MHz, CDCl₃) δ 13.6 (CH₃), 21.8 (CH₃), 22.3 (CH₂), 26.0 (CH₂), 27.7 (CH₂), 28.8 (CH₂), 31.8 (C), 37.3 (CH₂), 43.6 (CH₂), 72.3 (CH₂), 76.5 (C), 125.9 (CH-Ar), 134.4 (CH-Ar), 138.9 (C-*ipso*-Ar), 142.6 (C-*ipso*-Ar-B), 161.8 (C-*imidazole*), 186.6 (CO) ppm. IR (υ): 1725 (C=O), 1317 (B-O and C-O), 1133 (B-C) cm⁻¹. MS-Cl (NH₃): 397 (M⁺+1)

### Example 20: Preparation of 2-n-butyl-3-[{2'-[1-(triphenylmethyl)-1H-tetrazol-5-yl]-1,1'-biphenyl-4-yl}methyl]-1,3-diazaspiro[4.4]non-1-ene-4-one

A Schlenk tube was charged with 4-(2-n-butyl-4-spirocyclopentane-2-imidazolin-5-one-1-ylmethyl)benzeneboronic acid (150 mg), 5-(2-bromophenyl)-1-triphenylmethyl-1 H-tetrazole (182 mg), anhydrous K₂CO₃ (269 mg), toluene (2.5 mL), dimethylformamide (0.25 mL) and H₂O (0.8 mL). The suspension was purged and degassed. Dichloro[1,1-bis(diphenylphosphino)ferrocene]palladium (II) (PdCl₂(dppf)) was added, the mixture was purged and degassed. The reaction was heated at 85 °C for 48 h. After cooling to room temperature, ethyl acetate (2.6 mL) and H₂O (0.4 mL) were added, the mixture was stirred for 10 min. The organic layer was washed with H₂O (2.6 mL), dried over anhydrous Na₂SO₄, filtered and evaporated to dryness. The residue was purified by column chromatography (eluant: cyclohexane/ethyl acetate, 8:2) to yield 131 mg (50%).

### Example 21: Preparation of 2-n-butyl-3-[{2'-[1-(triphenylmethyl)-1H-tetrazol-5-yl]-1,1'-biphenyl-4-yl}methyl]-1,3-diazaspiro[4.4]non-1-ene-4-one

A Schlenk tube was charged with 2-n-butyl-3-[4-(5,5-dimethyl-[1,3,2]dioxaborinan-2-yl)benzyl]-1,3-diazaspiro[4.4]non-1-ene-4-one (127 mg), 5-(2-bromophenyl)-1-triphenylmethyl-1*H*-tetrazole (150 mg), anhydrous K₃PO₄ (203 mg) and anhydrous tetrahydrofuran (3.3 mL). The suspension was purged and degassed. Dichloro[1,1-bis(diphenylphosphino)ferrocene]-palladium (II) (PdCl₂(dppf)) was added, the mixture was purged and degassed. The reaction was refluxed for 48 h. After cooling to room temperature, the reaction mixture was filtered, the cake was washed with dichloromethane. The filtrate and washings were combined and evaporated to dryness. The residue was purified by column chromatography (eluant: cyclohexane/ethyl acetate, 8:2) to yield 68 mg (32%).

### Example 22: Preparation of 2-n-butyl-3-[{2'-[1-(triphenylmethyl)-1H-tetrazol-5-yl]-1,1'-biphenyl-4-yl}methyl]-1,3-diazaspiro[4.4]1non-1-ene-4-one

A Schlenk tube was charged with triphenylphosphine (4 mg) and anhydrous tetrahydrofuran (4 mL) and the resulting solution was purged and degassed. Palladium acetate (Pd(OAc)₂) was then added and the mixture was purged, degassed and stirred at room temperature for 30 min. Then, 2-n-butyl-3-[4-(5,5-dimethyl-[1,3,2]dioxaborinan-2-yl)benzyl]-1,3-diazaspiro[4.4]non-1-ene-4-one (150 mg), 5-(2-bromophenyl)-1-triphenylmethyl-1H-tetrazole (178 mg) and anhydrous K₃PO₄ (247 mg) were added. The suspension was purged and degassed, and the reaction was refluxed for 48 h. After cooling to room temperature, the reaction mixture was filtered, the cake was washed with dichloromethane and ethyl acetate. The filtrate and washings were combined and evaporated to dryness. The residue was purified by column chromatography (eluant: cyclohexane/ethyl acetate, 8:2) to yield 139 mg (54%). ¹H NMR (400 MHz, CDCl₃) δ 0.83 (t, *J* = 7.4 Hz, 3 H, CH₃), 1.25 (m, 2 H, CH₃-CH₂-CH₂-), 1.53 (qn, *J* = 7.6 Hz, CH₂-C*H*₂CH₂-), 1.82 (m, 2 H, CH₂), 1.99 (m, 6 H, CH₂), 2.24 (t, *J* = 7.8 Hz, CH₂-C*H*₂-C-). 4.57 (s, 2 H, Ph-C*H*₂-N-), 6.91-7.94 (m, 23 H, H-Ar) ppm. ¹³C NMR (100 MHz, CDCl₃) δ 13.9 (CH₃), 22.5 (CH₂), 26.3 (CH₂), 27.1 (CH₂), 27.9 (CH₂), 28.9 (CH₂), 37.6 (CH₂), 43.6 (CH₂), 76.7 (C), 83.1 (C), 126.5, 126.6, 127.9, 128.5, 130, 130.2, 130.4, 130.5 and 130.9 (CH-Ar), 135.3, 141, 141.4 and 141.7 (C-*ipso*-Ar), 164.2 (C-*imidazole*)*,* 186.8 (CO) ppm. IR (υ): 1724 (CO) cm⁻¹

### Example 23: Preparation of 2-n-butyl-3-{[2'-(1H-tetrazol-5-yl)-1,1'-biphenyl-4-yl]methyl}-1,3-diazaspiro[4.4]non-1-ene-4-one

A mixture of 2-n-Butyl-3-[{2'-[1-(triphenylmethyl)-1*H*-tetrazol-5-yl]-1,1'-biphenyl-4-yl}methyl]-1,3-diazaspiro[4.4]non-1-ene-4-one (139 mg) and 1,4-dioxane (1.7 mL), water (0.3 mL) and 4 N HCl (0.21 mL) was stirred at room temperature overnight. Water (72 mL) was added and the product was extracted with ethyl acetate (72 mL x 3). The combined organic layers were dried over anhydrous Na₂SO₄, filtered and the solvent was evaporated under reduced pressure. The residue was treated with diethyl ether and filtered. The solid was washed with cold diethyl ether and dried to obtain 2-*n*-butyl-3-{[2'-(1*H*-tetrazol-5-yl)-1,1'-biphenyl-4-yl]methyl}-1,3-diazaspiro[4.4]non-1-ene-4-one (56 mg, 62%). ¹H NMR (400 MHz, CDCl₃) δ 0.88 (t, J = 7.4 Hz, 3 H, CH₃), 1.34 (qn, J = 7.6 Hz, 2 H, CH₃-CH₂-CH₂-), 1.57 (t, J = 7.6 Hz, 2 H, CH₂-CH₂-CH₂-), 1.83 (m, 2 H, CH₂), 1.97 (m, 6 H, CH₂), 2.91 (bs, 2 H, CH₂-*CH*₂-C-), 4.69 (s, 2 H, Ph-C*H*₂-N-), 7.08 (d, *J* = 8 Hz, 2 H, H-Ar), 7.15 (d, *J* = 8.4 Hz, 2 H, H-Ar), 7.47 (d, *J* = 7.6 Hz, 1 H, H-Ar), 7.52 (td, *J* = 8.8 and 1.2Hz, 1 H, H-Ar), 7.61 (td, *J* = 7.6 and 1.2Hz, 1 H, H-Ar), 7.82 (dd, *J* = 7.6 and 0.8 Hz, 1 H, H-Ar) ppm. ¹³C NMR (100 MHz, CDCl₃) 13.3 (CH₃), 21.9 (CH₂), 25.8 (CH₂), 27.3 (CH₂), 37.2 (CH₂), 43.2 (CH₂), 76.1 (C), 126.5, 129.5, 127.6, 127.8, 127.9, 130.5, 130.6, 131, 135.4, 138.9, 140.9, 155.4 and 162.9 (CH-Ar and C-*ipso*-Ar), 186.5 (CO).IR (υ): 3450 (NH), 1733 (C=O) cm⁻¹. MS-ES (+): 671 (M⁺+1).

### Example 24: Preparation of ethyl 2-{[(5,5-dimethyl-[1,3,2]dioxaborinan-2-yl)methyl]amino}-3-nitrobenzoate

To a solution of ethyl 2-*tert*-butoxycarbonylamino-3-nitrobenzoate (0.15 g) in dimethylformamide (1ml) was added, while stirring under ice-cooling, sodium hydride (60% dispersion in mineral oil, 20 mg). The mixture was stirred at room temperature for 20 minutes and to the mixture was then added 2-(4-bromomethyl-phenyl]-5,5-dimethyl-[1,3,2]dioxaborinane (0.15 g) and potassium iodide (4 mg). The reaction mixture was stirred for 6h at 80 °C, followed by pouring into water (5 ml) and extracting with ethyl acetate (3x5 ml). The organic layers were combined, washed with water dried and concentrated to give a yellow syrup. The syrup was dissolved in a mixture of trifluoroacetic acid (0.17 ml) and methylene chloride (1.1 ml) and the solution was stirred for 1 hour and 30 minutes at room temperature. After dilution with methylene chloride (2 ml) the reaction mixture was washed with a saturated solution of sodium carbonate (2 ml) followed by water (2 ml), dried and evaporated to dryness to obtain a residue (0.20 g) which was chromatographed on silica (eluant: cyclohexane /ethyl acetate from 100/0 to 95/5) to obtain ethyl 2-{[(5,5-dimethyl-[1,3,2]dioxaborinan-2-yl)methyl]amino}-3-nitrobenzoate (0.11g, 57% yield) as a yellow solid. Mp: 91-95 °C. ¹H-NMR (400MHz, CDCl₃) δ 1.01 (s, 6H, C(CH₃)₂), 1.35 (t, J=7.2Hz, 3H, CH₃ of CO₂Et), 3.75 (s, 4H, 2CH₂O ), 4.15 (s, 2H, CH₂Ph), 4.32 (q, J=7.2Hz, 2H, CH₂ of CO₂Et), 6.69 (t, J=8 Hz, 1H, H-Ar), 7.27 (d, J=7.6 Hz, 2H, H-Ar), 7.76 (d, J=8 Hz, 2H, H-Ar), 7.98 (d, J=8 Hz, 1H, H-Ar), 8.08 (d, J=7.6 Hz, 1H, H-Ar), 8.79 (bm, 1H, NH) ppm.¹³C-NMR (100MHz, CDCl₃) δ 14.1 (CH₃, CH₃ of CO₂Et ), 21.8 (CH₃, CH₃ of C(CH₃)₂), 31.8 (C,C of C(CH₃)₂), 51.1 (CH₂), 61.4 (CH₂), 72.3 (CH₂), 114.6 (CH-Ar), 117.2 (C-Ar), 127.0 (CH-Ar), 131.4 (CH-Ar), 134.4 (CH-Ar), 136.8 (CH-Ar), 136.9 (C-Ar), 140.0 (C-Ar), 145.3 (C, C-Ar), 167.2 (C, CO) ppm. EM (Cl, NH₃): 413 (M⁺+1, 100), 412 (M+, 33).

### Example 25. Preparation of ethyl 3-amino-2-{ [(5,5-dimethyl-[1,3,2]dioxaborinan-2-yl)methyl]amino}benzoate

To a solution of ethyl 2-{ [(5,5-dimethyl-[1,3,2]dioxaborinan-2-yl)methyl]amino}-3-nitrobenzoate (0.52 g) in ethanol (10 ml) was added stannous dichloride dihydrate (1.14 g) and the mixture was stirred at 80°C for two hours. The solvent was evaporated to dryness. To the ice-cooling mixture of the residue in ethyl acetate (10 ml) was added dropwise 2N sodium hydroxyde (10 ml) with stirring. The aqueous layer was extracted with ethyl acetate (3x15 ml). The organic layers were combined, washed with water, and dried. The solvent was evaporated to dryness to obtain 0.42 g of ethyl 3-amino-2-{ [(5,5-dimethyl-[1,3,2]dioxaborinan-2-yl)methyl]amino}benzoate (88% yield) as a brown oil that was used in the next reaction without further purification.¹H-NMR (400MHz, CDCl₃) δ 1.00 (s, 6H, C(CH₃)₂), 1.29 (t, J=7.2Hz, 3H, CH₃ of CO₂Et), 3.75 (s, 4H, 2CH₂O) 4.16 (s, 2H, CH₂Ph), 4.22 (q, J=7.2 Hz, 2H, CH₂ of CO₂Et), 6.84 (m, 2H, H-Ar), 7.30 (m, 3H, H-Ar), 7.74 (d, J= 8 Hz, 2H, H-Ar) ppm.¹³C-NMR (100MHz, CDCl₃) δ 14.1 (CH₃, CH₃ of CO₂Et), 21.8 (CH₃, CH₃ of C(CH₃)₂), 31.8 (C, C of C(CH₃)₂ ), 50.3 (CH₂), 60.6 (CH₂), 72.2 (CH₂), 119.4 (CH-Ar), 120.7 (CH-Ar), 121.7 (C-Ar), 122.0 (CH-Ar), 127.0 (CH-Ar), 133.9 (CH-Ar), 134.5 (C-Ar), 138.9 (C-Ar), 141.4 (C-Ar), 142.6 (C-Ar), 168.2 (C, CO) ppm. EM (Cl, NH₃): 383 (M⁺+1, 100), 382 (M+, 34).

### Example 26: Preparation of ethyl 1-[(5,5-dimethyl-[1,3,2]dioxaborinan-2-yl)methyl]-2-ethoxybenzimidazole-7-carboxylate

A mixture of ethyl 3-amino-2-{[(5,5-dimethyl-[1,3,2]dioxaborinan-2-yl)methyl]amino}benzoate (80 mg), acetic acid (0.015 ml), and ethyl orthocarbonate (0.24 ml) in toluene (1 ml) was stirred at 80 °C for four hours. Ethyl acetate (2 ml) was added and the reaction mixture was washed with a saturated sodium hydrogen carbonate solution (1ml). Extraction was carried out with ethyl acetate (2x2 ml), followed by drying and evaporating to dryness under reduced pressure to give a residue (94 mg) which was chromatographed on silica (eluant:cyclohexane /ethyl acetate from 98/2 to 85/15) to obtain ethyl 1-[(5,5-dimethyl-[1,3,2]dioxaborinan-2-yl)methyl]-2-ethoxybenzimidazole-7-carboxylate (50mg, 55% yield) as a white solid. Mp: 117-120°C. ¹H-NMR (400MHz, CDCl₃) δ 0.98 (s, 6H, C(CH₃)₂), 1.21 (t, J=7.2Hz, 3H), 1.45 (t, J=7.2Hz, 3H) 3.71 (s, 4H, 2CH₂O), 4.18 (q, J=7.2 Hz, 2H), 4.64 (q, J=7.2 Hz, 2H), 5.63 (s, 2H, CH₂Ph) 6.93 (d, J=8Hz, 2H, H-Ar), 7.15 (t, J=8 Hz, 1H, H-Ar), 7.51 (d, J= 8 Hz, 1H, H-Ar), 7.64 (d, J= 8 Hz, 2H, H-Ar), 7.71 (d, J= 8 Hz, 1 H, H-Ar) ppm. ¹³C-NMR (100MHz, CDCl₃) δ 14.1 (CH₃), 14.6 (CH₃) 21.8 (CH₃, CH₃ of C(CH₃)₂), 31.8 (C, C of C(CH₃)₂ ), 47.2 (CH₂), 61.1 (CH₂), 66.6 (CH₂), 72.2 (CH₂), 116.2 (C-Ar), 120.7 (CH-Ar), 121.6 (CH-Ar), 123.4 (CH-Ar), 125.6 (CH-Ar), 131.4 (C-Ar), 134.0 (CH-Ar), 139.7 (C-Ar), 141.8 (C-Ar), 158.7 (C-Ar), 166.3 (C, CO) ppm. EM (Cl, NH₃): 437 (M⁺+1, 100), 436 (M⁺, 43).

### Example 27: Preparation of ethyl 2-ethoxy-1-{[2'-[1-(triphenylmethyl)-1H-tetrazol-5-yl]-1,1'-biphenyl-4-yl}methyl]-benzimidazole-7-carboxylate

A mixture of ethyl 1-[(5,5-dimethyl-[1,3,2]dioxaborinan-2-yl)methyl]-2-ethoxybenzimidazole-7-carboxylate (0.24 g), 5-(2-Bromophenyl)-1-triphenylmethyl-1 H-tetrazole (0.257 g) and K₃PO₄ (0.350 g) in anhydrous tetrahyrofuran (6 ml) was degassed by vacuum/nitrogen purges (3x). Then, dichloro [1,1'-bis(diphenylphosphino)ferrocene]palladium (II) (24 mg) was added, the mixture was degassed by vacuum/nitrogen purges (3x) and heated at reflux for 80 hours. The reaction mixture was cooled, passed through a pad of celite® and the filtrate evaporated under vacuum. The residue was chromatographed on silica (eluant: cyclohexane /ethyl acetate: from 98/2 to 70/30) to give ethyl 2-ethoxy-1-[{2'-[1-(triphenylmethyl)-1H-tetrazol-5-yl]-1,1'-biphenyl-4-yl}methyl]-benzimidazole-7-carboxylate (0.29 g, 74% yield) as a white solid. Mp: 158-159°C.¹H-NMR (400MHz, CDCl₃) δ 1.16 (t, J=7Hz, 3H), 1.41 (t, J=7.2Hz, 3H), 4.14 (q, J=7.2Hz, 2H), 4.62 (q, J=7Hz, 2H), 5.55 (s, 2H, CH₂Ph), 6.74 (d, J=8Hz, 2H, H-Ar), 6.92 (d, J=8.8Hz, 6H, H-Ar), 6.98 (d, J=8Hz, 2H, H-Ar), 7.15-7.35 (m, 11 H, H-Ar), 7.4 (m, 2H, H-Ar), 7.52 (d, J=7.6Hz, 1 H, H-Ar), 7.73 (d, J=8Hz, 1 H, H-Ar), 7.85 (d, J=7.4Hz, 1H, H-Ar) ppm. ¹³C-NMR (100MHz, CDCl₃) δ 14.1 (CH₃), 14.6 (CH₃), 46.8 (CH₂), 61.1 (CH₂), 66.6 (CH₂), 82.8 (C), 116.3 (C-Ar), 120.8 (CH-Ar), 121.7 (CH-Ar), 123.4 (CH-Ar), 125.9 (CH-Ar), 126.3 (C-Ar), 127.4 (CH-Ar), 127.6 (CH-Ar), 128.1 (CH-Ar), 129.3 (CH-Ar), 129.8 (CH-Ar), 130.2 (CH-Ar), 130.3 (CH-Ar), 130.6 (CH-Ar), 131.3 (C-Ar), 135.8 (C-Ar), 139.9 (C-Ar), 141.2 (C-Ar), 141.7 (C-Ar), 158.6 (C-Ar), 164.0 (C-Ar), 166.3 (C-Ar) ppm.

### Example 28: Preparation of 2-ethoxy-1-{[2'-(1H-tetrazol-5-yl)-1,1'-biphenyl-4-yl]methyl}-benzimidazole-7-carboxylic acid

A solution of ethyl 2-ethoxy-1-[{2'-[1-(triphenylmethyl)-1*H*-tetrazol-5-yl]-1,1'-biphenyl-4-yl}methyl]-benzimidazole-7-carboxylate (0.28g) and 1M LiOH (1.2 ml) in ethanol (3.5 ml) was stirred at 80°C for three hours. The reaction mixture was concentrated and washed with toluene (5x2ml). The aqueous layer was adjusted to pH 2-3 with 1 M HCl to give a white solid (0.15g, 85% yield) corresponding to 2-ethoxy-1-{[2'-(1H-tetrazol-5-yl)-1,1'-biphenyl-4-yl]methyl}-benzimidazole-7-carboxylic acid. ¹H-NMR (400MHz, DMSO) δ 1.37 (t, J=7Hz, 3H), 4.57 (q, J=7Hz, 2H), 5.62 (s, 2H), 6.95 (q, 4H), 7.17 (t, J=8Hz, 1H), 7.46 (m, 1 H), 7.5 (m, 2H), 7.6-7.7 (m, 3H) ppm.

### Example 29: Preparation of 2-ethoxy-1-{[2'-[1-(triphenylmethyl)-1H-tetrazol-5-yl]-1,1'-biphenyl-4-yl}methyl]-benzimidazole-7-carboxylic acid

To a solution of 2-ethoxy-1-{[2'-(1H-tetrazol-5-yl)-1,1'-biphenyl-4-yl]methyl}-benzimidazole-7-carboxylic acid (78 mg) in methylene chloride (1ml) were added trityl chloride (63 mg) and triethylamine (0.03ml). The mixture was stirred at room temperature for two hours thirty minutes. The reaction mixture was washed with water, dried and concentrated to dryness. The residue was purified by column chromatography on silica (eluant: cyclohexane /ethyl acetate: from to 90/10 to 0/100) to give 2-ethoxy-1-[{2'-[1-(triphenylmethyl)-1*H*-tetrazol-5-yl]-1,1'-biphenyl-4-yl}methyl]-benzimidazole-7-carboxylic acid (60mg, 53% yield) as a white solid. ¹H-NMR (400MHz, CDCl₃) δ 1.40 (t, J=7Hz, 3H), 4.62 (q, J=7Hz, 2H), 5.59 (s, 2H), 6.76 (d, J=8Hz, 2H), 6.92-6.96 (m, 8H), 7.14 (t, J=8Hz, 1 H), 7.19-7.30 (m, 10H), 7.38 (m, 2H), 7.64 (d, J=8Hz, 1 H), 7.76-7.83 (m, 2H) ppm.

## Claims

1. A compound of formula (II), 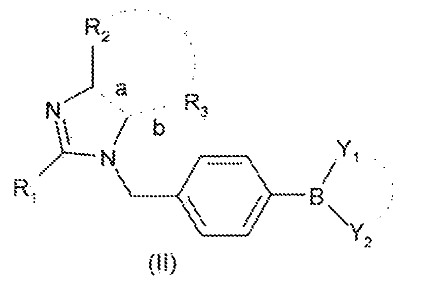 wherein:
Y₁ and Y₂ are each independently selected from the group consisting of hydroxy. (C₁-C₄)-alcoxy and phenoxy, the latter optionally substituted by a (C₁-C₄)-alcoxy, (C₁-C₄)-alkyl or an halogen group; or alternatively Y₁ and Y₂ can be taken together with the boron atom to form a cyclic structure selected from the following ones, 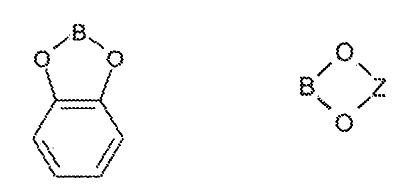
wherein Z is selected from the group consisting of (CH₂)ₙ, (CH₂)ᵣCRₐR_{y}(CH₂)ₛ and CRₐR_{y}(CH₂)ₜCRᵤRᵥ is an integer from 2 to 4: r and s are integers from 0 to 4 with the condition that r and s are not both 0; t is an integer from 0 to 1, and Rₐ and Rᵥ are each independently selected from the group consisting of H, (C₁-C₄)-alkyl, phenyl and mono- or di- substituted phenyl, the substituents being halogen, (C₁-C₄)-alkyl and (C₁-C₄)-alkoxy;
a and b are single or double bonds,
R₁ is a radical selected from (C₁-C₄)-alkyl and (C₁-C₄)-alkoxy;
R₂ is or alternatively R₂ forms a spiro-fused ring from 4 to 6 carbon atoms with the carbon atom of the heterocyclic ring; R₃ is O or a radical selected from the group consisting of hydroxymethyl and formyl, which are optional protected; or alternatively, R₂ and R₃ are bound together forming with the heterocyclic ring a fused benzene ring substituted by a radical selected from the group consisting of (C₁-C₈)-alkoxycarbonyl and 1-[[(cyclohexyloxy)carbonyl]oxy]ethoxycarbonyl;
with the condition that.
(i) when a is a single bond, b is a double bond. R₂ forms a ring from 4 to 6 carbon atoms with the carbon atom of the heterocyclic ring and R₃ is O; and
(ii) when a is a double bond, b is a single bond, R₂ is Cl; and R₃ is a radical selected from the group consisting of hydroxymethyl and formyl, which are optionally protected; or alternatively, R₂ and R₃ are bound together forming with the heterocyclic ring a fused benzene ring, substituted by a radical selected from the group consisting of (C₁-C₆)-alkoxycarbonyl and 1.{[(cyclohexyloxy)carbonyl]oxy}ethoxycarbonyl.

2. The compound according to claim 1, wherein R₁ is butyl, R₂ forms a spiro-fused ring of 5 carbon atoms with the carbon atom of the heterocyclic ring, a is a single bond, b is a double bond and R₃ is O.

3. The compound according to claim 1, wherein R₁ is butyl, R₂ is Cl, a is a double bond, b is a single bond and R₃ is a hydroxymethyl or a formyl radical.

4. The compound according to claim 1, wherein R₁ is a (C₃-C₄)-alkoxy radical; a is a double bond; and R₂ and R₃ are bound together forming with the heterocyclic ring a fused benzene ring substituted by a radical selected from the group consisting of (C₁-C₆)-alkoxycarbonyl and 1-{[(cyclohexyloxy)carbonyl]oxy}-ethoxycarbonyl.

5. The compound according to claim 4, wherein R₁ is an ethoxy radical, and R₂ and R₃ are bound together forming with the heterocyclic ring a fused benzene ring substituted by a ethoxycarbonyl radical.

6. The compound according to claim 4, wherein R₁ is an ethoxy radical, and R₂ and R₃ are bound together forming with the heterocyclic ring a fused benzene ring substituted by a methoxycarbonyl radical.

7. The compound according to any of the claims 1-6, wherein Y₁ and Y₂ are independently selected from the group consisting of: hydroxy, methoxy, ethoxy and phenoxy, or alternatively, Y₁ and Y₂ together with the boron atom form a cyclic structure, wherein Z is selected from the group consisting or (CH₂)ₜCRᵤRᵥ(CH₂)₅ and CRᵤRᵥ(CH₂)ₜCRᵤRᵥ; r and s are integers from 0 to 4 with the condition that r and s are not both 0; t is an integer from 0 to 1 and Rᵤ and Rᵥ are each independently selected from methyl and phenyl.

8. The compound according to claim 7, wherein Y₁ and Y₂ are hydroxy.

9. The compound according to claim 7, wherein Y₁ and Y₂ together with the boron atom form a cyclic structure, wherein Z is CH₂C(CH₃)₂CH₂.

10. The compound according to claim 7, wherein Y₁ and Y₂ together with the boron atom form a cyclic structure, wherein Z is C(CH₃)₂C(CH₃)₂.

11. The compound according to claim 1, which is selected from the group consisting of :
4-(2-n-butyl-4-chloro-5-formylimidazol-1-ylmethyl)benzeneboronic acid;
2-n-butyl-4-chloro-1-[4-(5,5-dimethyl-[1,3,2]dioxaborinan-2-yl)benzyl]-1H-imidazole-5-carbaldehyde ;
4-(2-n-butyl-4-chloro-5-hydroxymethylimidazol-1-ylmethyl)benzeneboromic acid;
2-n-butyl-4-chloro-1-[4-(5,5-dimethyl-[1,3,2]dioxaborinan-2-yl)benzyl]-1H-imidazole-5-methanol;
4-(2-n-butyl-4-spirocyclopentane-2-imidazolin-5-one-1-ylmethyl)benzeneboronic acid;
2-n-butyl-3-[4-(5,5-dimethyl-[1,3,2]dioxaborinan-2-yl)benzyl]-1,3-diazaspiro[4,4]non-1-ene-4-one;
ethyl 1-[(5,5-dimethyl-[1,3,2]dioxaborinan-2-yl)methyl]-2-ethoxybenzimidazole-7-carboxylate;
methyl 1-[(5,5-dimethyl-[1,3,2]dioxaborinan-2-yl)methyl]-2-ethoxybenzimidazole-7-carboxylate; and
2-n-butyl-4-chloro-1-[4-(4,4,5,5-tetramethyl-[1,3,2]dioxoborolan-2-yl)benzyl]-1H-imidazole-5-carbaldehyde.

12. A preparation process of a compound of formula (II) as defined in any of the claims 1-11, comprising a reaction of a compound of formula (III), 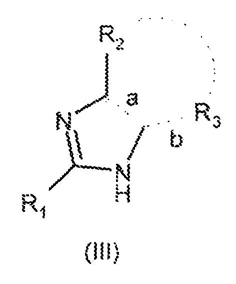 with a boron compound of formula (IV) 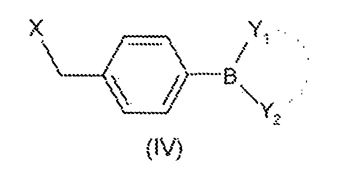 in the presence of a base and an appropriate solvent,
wherein X is a leaving group; and each of R₁, R₂, R₃, Y₁ and Y₂ is a group as defined in claim 1, or an intermediate or protected form thereof which can be transformed to such a group, and thereafter as necessary transforming said intermediate or protected forms of the R₁, R₂ ,R₃, Y₁ and Y₂ groups to R₁, R₂, R₃, Y₁ and Y₂ groups as defined in claim 1.

13. The preparation process according to claim 12, wherein the base is an inorganic base selected from the group consisting of alkaline metal hydride, alkaline metal hydroxide, alkaline metal carbonate and metal alkoxyde.

14. The preparation process according to any of the claims 12-13, wherein the leaving group X is Br.

15. preparation process of a compound as defined in any of the claims 4-6, comprising the steps of :
(a) reacting a compound of formula (V) 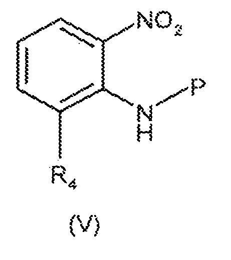 with the boron compound or formula (IV), 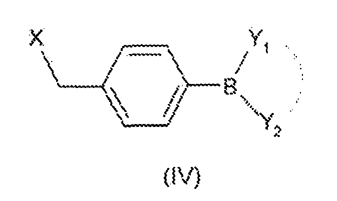 in the presence of a base and an appropriate solvent; followed by the cleavage of the protective amino group P and the reduction of the nitro group to yield a compound of formula (VI); and 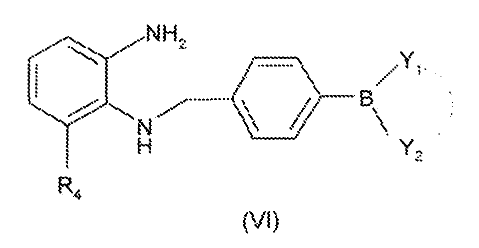
(b) treating the compound (VI) with alkyl orthocarbonate or a carbonylating agent, to give a compound of formula (II'); 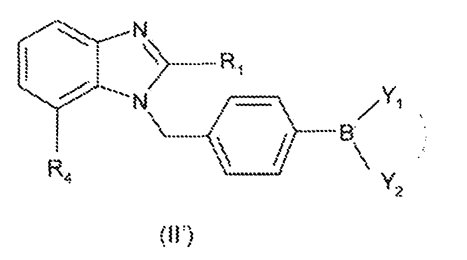
wherein P is an amino protective group; X is a leaving group; R₄ is a radical selected from the group consisting of (C₁-C₆)-alkoxycarbonyl and 1-{[(cyclohexyloxy)carbonyl]oxy}-ethoxycarbonyl; Y₁ and Y₂ are each independently selected from the group consisting of hydroxy, (C₁-C₄)-alcoxy and phenoxy, the latter optionally substituted by a (C₁-C₄)-alcoxy, (C₁-C₄)-alkyl or an halogen group; or alternatively Y₁ and Y₂ can be taken together with the boron atom to form a cyclic structure selected the following ones, 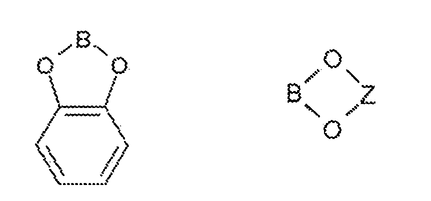 wherein Z is selected from the group consisting of (CH₂)ₙ, (CH₂)ₜCRᵤRᵥ(CH₂)ₛ and CRᵤRᵥ(CH₂)ₜCRᵤRᵥ; n is an integer from 2 to 4; r and s are integers from 0 to 4 with the condition that r and s are not both 0; t is an integer from 0 to 1, and Rᵤ and Rᵥ are each independently selected from the group consisting of H, (C₁-C₄)-alkyl, phenyl and mono- or di- substituted phenyl, the substituents being halogen, (C₁-C₄)-alkyl and (C₁-C₄)-alkoxy; and each of R₄ R₁, Y₁ and Y₂ is a group as defined above, or an intermediate or protected form thereof which can be transformed to such a group, and thereafter as necessary transforming said intermediate or protected forms of the R₄, R₁, Y₁ and Y₂ groups to R₄, R₁, Y₁ and Y₂ groups as defined above.

16. The preparation process according to claim 15, wherein Y₁ and Y₂ are hydroxy.

17. The preparation process according to claim 15, wherein Y₁ and Y₂ together with the boron atom form a cyclic structure, wherein Z is CH₂C(CH₃)₂CH₂.

18. The preparation process according to claim 15, wherein Y₁ and Y₂ together with the boron atom form a cyclic structure, wherein Z is C(CH₃)₂C(CH₃)₂.

19. The preparation process according to any of the claims 15-18, wherein the leaving group X is Br.

20. A preparation process of a compound of formula (I), or a pharmaceutical salt thereof, 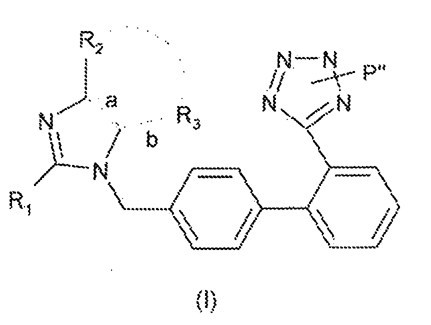 wherein:
a and b are single or double bonds,
P" is H or the protective group P';
R₁ is a radical selected from (C₁-C₄)-alkyl and (C₁-C₄)-alkoxy;
R₂ is Cl; or alternatively R₂ forms a spire-fused ring from 4 to 6 carbon atoms with the carbon atom of the heterocyclic ring. R₃ is O or a radical selected from the group consisting of hydroxymethyl and formyl, which are optionally protected; or alternatively, R₂ and R₃ are bound together forming with the heterocyclic ring a fused benzene ring substituted by a radical selected from the group consisting of carboxyl, (C₁-C₆)-alkoxycarbonyl and 1-{[(cyclohexyloxy)carbonyl]oxy}ethoxycarbonyl;
with the condition that:
(i) when a is a single bond, b is a double bond, R₂ forms a spiro-fused ring from 4 to 6 carbon atoms with the carbon atom of the heterocyclic ring and R₃ is O; and
(ii) when a is a double bond, b is a single bond, R₂ is Cl, and R₃ a radical selected from the group consisting of hydroxymethyl and formyl, which are optionally protected; or alternatively, R₂ and R₃ are bound together forming with the heterocyclic ring a fused benzene ring, substituted by a radical selected from the group consisting of carboxyl, (C-₃-C₈)-alkoxycarbonyl and 1-{[(cyclohexyloxy)carbonyl]oxy}-ethoxycarbonyl;
which comprises a coupling reaction of a compound of formula (II), as defined in any of the claims 1-11, 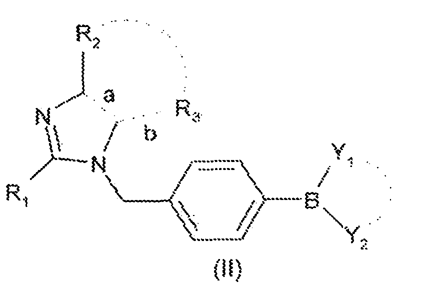 with a compound of formula (VII) 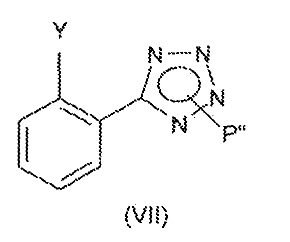 in an appropriate solvent system and in the presence of a metallic compound and a base;
wherein P" is H or a protective group P'; Y is a leaving group: and each of R₁, R₂ and R₃ is a group as defined in claim 1, or an intermediate or protected form thereof which can be transformed to such a group, and thereafter as necessary transforming said intermediate or protected forms of the R₁, R₂ and R₃ groups to R₁, R₂ and R₃ groups as defined in claim 1; and optionally when P" is a protective group P' submitting the compound obtained to a deprotection and optionally the process includes an additional step of converting the reaction product to salt form.

21. The preparation process according to claim 20, wherein P" is a protective group P'.

22. The preparation process according to any of the claims 20-21, wherein the leaving group Y is Br.

23. The preparation process according to any of the claims 20-22, wherein R₁ is butyl, R₂ form a spiro-fused ring of five carbon atoms with the carbon atom of the heterocyclic ring, a is a single bond, b is a double bond, R₃ is O and P" is H.

24. The preparation process according to any of the claims 20-22, wherein R₁ is butyl, R₂ is Cl, a is a double bond, b is a single bond, R₃ is hydroxymethyl and P" is H.

25. The preparation process according to any of the claims 20-22, wherein R₁ is butyl, R₂ is Cl, a is a double bond, b is a single bond, R₃ is and P" is trityl,

26. The preparation process according to any of the claims 20-22, wherein R₁ is a (C₁-C₄)-alkoxyl radical; a is a double bond; R₂ and R₃ are bound together forming with the heterocyclic ring a fused benzene ring substituted by a radical selected from the group consisting of carboxyl, (C₁-C₈)-alkoxycarbonyl and 1-{[(cyclohexyloxy)carbonyl]oxyl}ethoxycarbonyl, and P" is H or trityl.

27. The preparation process according to claim 26, wherein R₁ is a ethoxy radical, R₂ and R₃ are bound together forming with the heterocyclic ring a fused benzene ring substituted by a carboxyl radical, and P" is H.

28. The preparation process according to claim 26, wherein R₁ is a ethoxy radical, R₂ and R₃ are bound together forming with the heterocyclic ring a fused benzene ring substituted by a ethoxycarbonyl radical, and P" is trityl,

29. The preparation process according to claim 26, wherein R₁ is a ethoxy radical, R₂ and R₃ are bound together forming with the heterocyclic ring a fused benzene ring substituted by a methoxycarbonyl radical, and P" is trityl.

30. The preparation process according to claim 26, wherein R₁ is a ethoxy radical, R₂ and R₃ are bound together forming with the heterocyclic ring a fused benzene ring substituted by a 1-{[(cyclohexyloxy)carbonyl]oxy}ethoxycarbonyl radical, and P" is H.

31. The preparation process according to any of the claims 20-30, wherein the metallic compound is selected from palladium, nickel, a metallic salt and a metallic complex,

32. A compound of formula (VI), 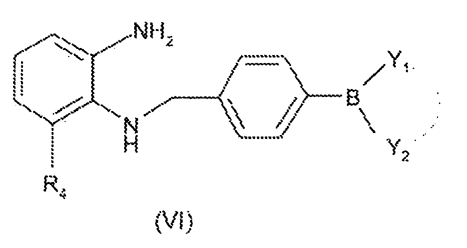 wherein;
R₄ is a radical selected from the group consisting (C₁-C₆)-alkoxycarbonyl and 1-{[(cydohexyloxy)carbonyl]oxy}-ethoxycabonyl; Y₁ and Y₂ are each independently selected from the group consisting of hydroxy, (C₁-C₄)-alcoxy and phenoxy, the latter optionally substituted by a (C₁-C₄)-alcoxy, (C₁-C₄)-alkyl or an halogen group: or alternatively Y₁ and Y₂ can be taken together with the boron atom to form a cyclic structure selected from the following ones, 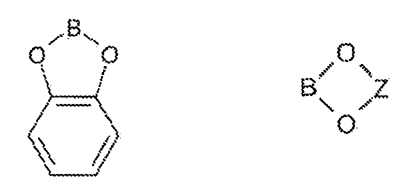 wherein Z is selected from the group consisting of (CH₂)ₙ, (CH₂)ᵣCRᵤRᵥ(CH₂)ₛ and CRᵤRᵥ(CH₂)ₜCRᵤRᵥ; n is an integer from 2 to 4; r and s are integers from 0 to 4 with the condition that r and s are not both 0; t is an integer from 0 to 1, and Rᵤ and Rᵥ are each independently selected from the group consisting of H, (C₁-C₄)-alkyl, phenyl and mono- or di- substituted phenyl, the substituents being halogen, (C₁-C₄)-alkyl and (C₁-C₄)-alkoxy; and each of R₄, Y₁ and Y₂ is a group as defined above, or an intermediate or protected form thereof which can be transformed to such a group, and thereafter as necessary transforming said intermediate or protected forms of the R₄, R₁, Y₁ and Y₂ groups to R₄, R₁, Y₁ and Y₂ groups as defined above.

33. Ethyl 3-amino-2-{[(5,5-dimethyl-[1,3,2]dioxaborinan-2-yl)methyl]amino} benzoate.

## Patentansprüche

1. Eine Verbindung der Formel (II), 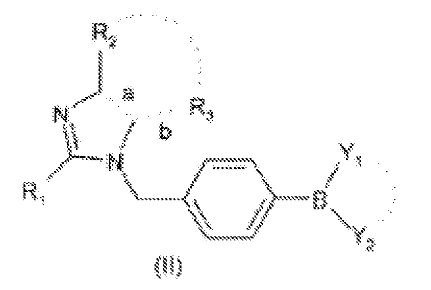 worin:
Y₁, und Y₂ unabhängig voneinander ausgewählt sind aus der Gruppe bestehend aus Hydroxy, (C₁-C₄)-Alkoxy und Phenoxy, wobei letztere optional durch eine (C₁-C₄)-Alkoxy-, (C₁-C₄)-Alkyl- oder eine Halogengruppe substituiert wird; oder alternativ können Y₁ und Y₂ mit dem Boratom zusammen genommen werden, um eine zyklische Struktur zu bilden, die aus folgendem ausgewählt wird: 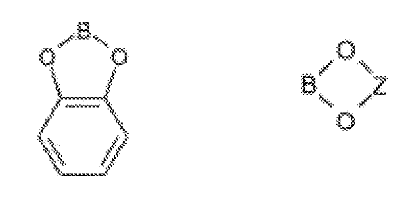 worin Z ausgewählt ist aus der Gruppe bestehend aus (CH₂)ₙ, (CH₂)ᵣCRᵤRᵥ(CH₂)ₛ und CRᵤRᵥ(CH₂)ₜCRᵤRᵥ; n ist eine ganze Zahl von 2 bis 4, r und s sind ganze Zahlen von 0 bis 4 unter der Bedingung, dass r und s nicht beide 0 sind, t ist eine ganze Zahl von 0 bis 1, und Rᵤ und Rᵥ sind unabhängig voneinander ausgewählt aus der Gruppe bestehend aus H, (C₁-C₄)-Alkyl, Phenyl und mono- oder di-substituiertem Phenyl, wobei die Substituenten Halogen, (C₁-C₄)-Alkyl und (C₁-C₄)-Alkoxy sind;
a und b sind einzelne oder doppelte Verbindungen,
R₁ ist ein Radikal, das aus (C₁-C₄)-Alkyl und (C₁-C₄)-Alkoxy ausgewählt ist;
R₂ ist Cl, oder alternativ bildet R₂ einen spiroanellierten Ring von 4 bis 6 Kohlenstoffatomen mit dem Kohlenstoffatom des heterocyclischen Ringes; R₃ ist O oder ein Radikal ausgewählt aus der Gruppe bestehend aus Hydroxymethyl und Formyl, welche optional geschützt sind; oder alternativ sind R₂ und R₃ gebunden und bilden zusammen mit dem heterocyclischen Ring einen anellierten Benzolring, der substituiert ist durch ein Radikal ausgewählt aus der Gruppe bestehend aus (C₁-C₆)-Alkoxycarbonyl und 1-{[(Cyclohexyloxy)carbonyl]oxy]ethoxycarbonyl;
mit der Bedingung, dass
(i) wenn a eine einfache Verbindung ist, so ist b eine doppelte Verbindung, R₂ bildet einen spiro-anellierten Ring von 4 bis 6 Kohlenstoffatomen mit dem Kohlenstoffatom des heterocyclischen Ringes und R₃ ist O; und
(ii) wenn a eine doppelte Verbindung ist, so ist b eine einfache Verbindung, R₂ ist Cl; und R₃ ist ein Radikal, das ausgewählt ist aus der Gruppe bestehend aus Hydroxymethyl und Formyl, welche optional geschützt sind oder alternativ sind R₂ und R₃ gebunden und bilden zusammen mit dem heterocyclischen Ring einen anellierten Benzolring, der durch ein Radikal substituiert ist, das aus der Gruppe bestehend aus (C₁-C₆)-Alkoxycarbonyl und 1-{[(Cyclohexyloxy)carbonyl]oxy} Ethoxycarbonyl ausgewählt ist.

2. Die Verbindung nach Anspruch 1, worin R₁ Butyl bedeutet, R₂ bildet einen spiro-anellierten Ring von 5 Kohlenstoffatomen mit dem Kohlenstoffatom des heterocyclischen Ringes, a bedeutet eine einfache Verbindung, b bedeutet eine doppelte Verbindung und R₃ ist O.

3. Die Verbindung nach Anspruch 1, worin R₁ Butyl bedeutet, R₂ ist Cl, a ist eine doppelte Verbindung, b ist eine einfache Verbindung und R₃ ist ein Hydroxymethyl- oder ein Formyl-Radikal.

4. Die Verbindung nach Anspruch 1, worin R₁ ein (C₁-C₄)-Alkoxyradikal ist; a bedeutet eine doppelte Verbindung und R₂ und R₃ sind gebunden und bilden zusammen mit dem heterocyclischen Ring einen anellierten Benzolring, der durch ein Radikal substituiert ist, das aus der Gruppe bestehend aus (C₁-C₆)-Alkoxycarbonyl und 1-{[(Cyclohexyloxy)carbonyl]oxy}-ethoxycarbonyl ausgewählt ist.

5. Die Verbindung nach Anspruch 4, worin R₁ ein Ethoxyradikal ist, und R₂ und R₃ sind gebunden und bilden zusammen mit dem heterocyclischen Ring einen anellierten Benzolring, der durch ein Ethoxycarbonyl-Radikal substituiert ist.

6. Die Verbindung nach Anspruch 4, worin R₁ ein Ethoxyradikal ist, und R₂ und R₃ sind gebunden und bilden zusammen mit dem heterocyclischen Ring einen anellierten Benzolring, der durch ein Methoxycarbonyl-Radikal substituiert ist.

7. Die Verbindung gemäß einem der Ansprüche 1 - 6, worin Y₁ und Y₂ unabhängig voneinander ausgewählt sind aus der Gruppe bestehend aus Hydroxy, Methoxy, Ethoxy und Phenoxy, oder alternativ Y₁ und Y₂ zusammen mit dem Boratom eine zyklische Struktur bilden, worin Z ausgewählt ist aus Gruppe bestehend aus (CH₂)ᵣ,CRᵤRᵥ(CH₂)ₛ und CRᵤRᵥ(CH₂)ₜCRᵤRᵥ; r und s sind ganze Zahlen von 0 bis 4 mit der Bedingung, dass r und s nicht beide 0 sind. t ist eine ganze Zahl aus 0 bis 1 und Rᵤ und Rᵥ sind unabhängig voneinander ausgewählt aus Methyl und Phenyl.

8. Die Verbindung nach Anspruch 7, worin Y₁ und Y₂ Hydroxy bedeuten.

9. Die Verbindung nach Anspruch 7, worin Y₁ und Y₂ zusammen mit dem Boratom eine zyklische Struktur bilden, worin Z ist CH₂C(CH₃)₂CH₂.

10. Die Verbindung nach Anspruch 7, worin Y₁ und Y₂ zusammen mit dem Boratom eine zyklische Struktur bilden, worin Z ist C(CH₃)₂C(CH₃)₂.

11. Die Verbindung nach Anspruch 1, welche aus folgender Gruppe ausgewählt ist:
4-(2-n-Butyl-4-chlor-5-formylimidazol-1-ylmethyl)benzolborsäure;
2-n-Butyl-4-chlor-1-[4-(5,5-dimethyl-[1,3,2]dioxaborinan-2-yl)benzyl]-1H-Imidazol-5-carbaldehyd;
4-(2-n-Butyl-4-chlor-5-hydroxymethylimidazol-1-ylmethyl)benzolborsäure;
2-n-Butyl-4-chlor-1-[4-(5,5-dimethyl-(1,3,2)dioxaborinan-2-yl)benzyl)-1H-imidazol-5-methanol;
4-(2-n-Butyl-4-spirocyclopentan-2-imidazol in-5-on-1-ylmethyl)benzolborsäure;
2-n-Butyl-3-[4-(5,5-dimethyl-[1,3,2]dioxaborinan-2-yl)benzyl]-1,3-diazaspiro[4,4]non-1-en-4-on;
Ethyl 1-[(5,5-dimethyl-[1,3,2]dioxaborinan-2-yl)methyl]-2-ethoxybenzimidazol-7-carboxylat;
Methyl 1-[(5,5-dimethyl-[1,3,2]dioxaborinan-2-yl)methyl]-2-ethoxybenzimidazol-7-carboxylat; und
2-n-Butyl-4-chlor-1-[4-(4,4,5,5-tetramethyl-[1,3,2]dioxoborolan-2-yl)benzyl]-1H-imidazol-5-carbaldehyd.

12. Ein Vorbereitungsvorgang einer Verbindung der Formel (II) nach einem der Ansprüche 1 - 11, der eine Reaktion einer Verbindung der Formel (III) umfasst, 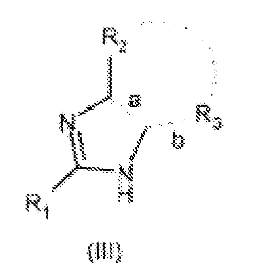 mit einer Borverbindung der Formel (IV) 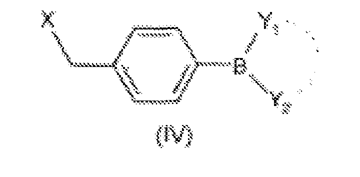 in Gegenwart von einer Basis und einem geeigneten Lösungsmittel:
worin X eine Abgangsgruppe ist und jede der Gruppen R₁, R₂, R₃, Y₁ und Y₂ eine Gruppe nach Anspruch 1 ist oder eine Zwischenform oder eine geschützte Form davon, die in eine solche Gruppe umgewandelt werden kann, und danach nach Notwendigkeit die besagten Zwischen- oder geschützten Formen der Gruppen R₁, R₂, R₃, Y₁ und Y₂ in die Gruppen R₁, R₂, R₃, Y₁ und Y₂, wie im Anspruch 1 festgelegt, umwandelt.

13. Der Vorbereitungsvorgang nach Anspruch 12, worin die Basis eine anorganische Basis ist, die ausgewählt ist aus der Gruppe bestehend aus Alkalimetallhydrid, Alkalimetallhydroxid, Alkalimetallkarbonat und Metallalkoxyd.

14. Der Vorbereitungsvorgang gemäß einem der Ansprüche 12-13, worin die Abgangsgruppe X ist Br.

15. Ein Vorbereitungsvorgang einer Verbindung nach einem der Ansprüche 4 - 6, der folgende Etappen umfasst:
(a) Reaktion einer Verbindung der Formel (V) 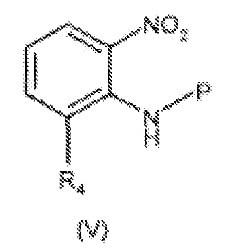 mit der Borverbindung der Formel (IV), 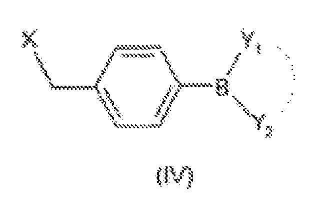 in Gegenwart von einer Basis und einem geeigneten Lösungsmittel, gefolgt von der Spaltung der Schutz- Aminogruppe P und der Reduktion der Nitrogruppe, um eine Verbindung der Formel (VI) zu ergeben; und 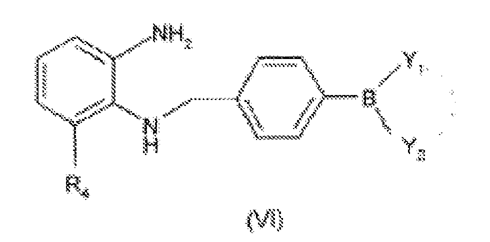
(b) Behandlung der Verbindung (VI) mit Alkylorthocarbonat oder einem Carbonylierungsmittel, um eine Verbindung der Formel (II') zu erhalten; 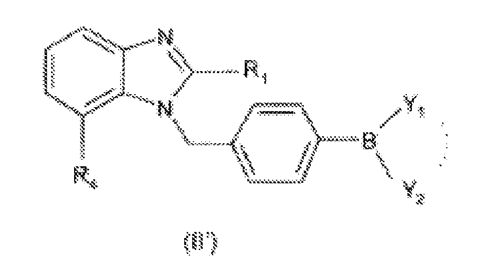
worin P eine Amino-Schutzgruppe ist, X eine Abgangsgruppe ist, R₄ ein Radikal ist, das aus der Gruppe bestehend aus (C₁-C₆)-Alkoxycarbonyl und 1-{[(Cyclohexyloxy)carbonyl]oxy)-ethoxycarbonyl ausgewählt ist; Y₁ und Y₂ sind unabhängig voneinander ausgewählt aus der Gruppe bestehend aus Hydroxy, (C₁-C₄)-Alkoxy und Phenoxy, wobei letztere optional durch eine (C₁-C₄)-Alkoxygruppe, (C₁-C₄)-Alkylgruppe oder eine Halogengruppe substituiert ist; oder alternativ können Y₁ und Y₂ zusammen mit dem Boratom genommen werden, um eine zyklische Struktur zu bilden, die aus folgendem ausgewählt ist, 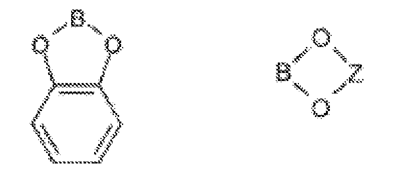 worin Z ausgewählt ist aus einer Gruppe bestehend aus (CH₂)ₙ, (CH₂)ᵣCRᵤRᵥ(CH₂)ₛ und CRᵤRᵥ(CH₂)ₜCRᵤRᵥ; n ist eine ganze Zahl von 2 bis 4; r und s sind ganze Zahlen von 0 bis 4, mit der Bedingung, dass r und s nicht beide 0 sind; t ist eine ganze Zahl von 0 bis 1 und Rᵤ und Rᵥ sind unabhängig voneinander ausgewählt aus der Gruppe bestehend aus H, (C₁-C₄)-Alkyl, Phenyl und mono- oder di-substituiertem Phenyl, wobei die Substituenten Halogen, (C₁-C₄)-Alkyl und (C₁-C₄)-Alkoxy sind; und jede der Gruppen R₄, R₁, Y₁ und Y₂ ist eine Gruppe nach der obigen Definition oder eine Zwischen- oder geschützte Form davon, die in eine solche Gruppe umgewandelt werden kann, und danach werden nach Bedarf die besagten Zwischen- oder geschützten Formen der Gruppen R₄, R₁, Y₁ und Y₂ in die Gruppen R₄, R₁, Y₁ und Y₂ nach der obigen Definition umwandelt.

16. Der Vorbereitungsvorgang nach Anspruch 16, worin Y₁ und Y₂ Hydroxy sind.

17. Der Vorbereitungsvorgang nach Anspruch 15, worin Y₁ und Y₂ zusammen mit dem Boratom eine zyklische Struktur bilden, worin Z = CH₂C(CH₃)₂CH₂.

18. Der Vorbereitungsvorgang nach Anspruch 15, worin Y₁ und Y₂ zusammen mit dem Boratom eine zyklische Struktur bilden, worin Z = C(CH₃)₂C(CH₃)₂.

19. Der Vorbereitungsvorgang gemäß einem der Ansprüche 15 - 18, worin die Abgangsgruppe X ist Br.

20. Ein Vorbereitungsvorgang einer Verbindung der Formel (1), oder ein pharmazeutisches Salz davon, 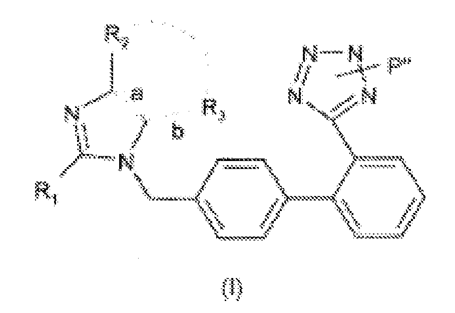 worin:
a und b einzelne oder doppelte Verbindungen sind,
P" ist H oder die Schutzgruppe P';
R₁ ist ein aus (C₁-C₄)-Alkyl und (C₁-C₄)-Alkoxy ausgewähltes Radikal:
R₂ ist Cl, oder alternativ bildet R₂ einen spiro-anellierten Ring von 4 bis 6 Kohlenstoffatomen mit dem Kohlenstoffatom des heterocyclischen Ringes; R₃ ist O oder ein aus der Gruppe bestehend aus Hydroxymethyl und Formyl ausgewähltes Radikal, das optional geschützt ist oder alternativ sind R₂ und R₃ gebunden und bilden zusammen mit dem heterocyclischen Ring einen anellierten Benzolring, der durch ein Radikal substituiert ist, das ausgewählt ist aus der Gruppe bestehend aus Carboxyl, (C₁-C₆)-Alkoxycarbonyl und 1-{[(Cyclohexyloxy)carbonyl)oxy} Ethoxycarbonyl:
mit der Bedingung, dass:
(i) wenn a eine einfache Verbindung ist, so ist b eine doppelte Verbindung, R₂ bildet einen spiro-anellierten Ring von 4 bis 6 Kohlenstoffatomen mit dem Kohlenstoffatom des heterocyclischen Ringes und R₃ ist O; und
(ii) wenn a eine doppelte Verbindung ist, so ist b eine einfache Verbindung, R₁ ist Cl, und R₃ ein Radikal, das ausgewählt ist aus der Gruppe bestehend aus Hydroxymethyl und Formyl, die optional geschützt sind oder alternativ sind R₂ und R₃ gebunden und bilden zusammen mit dem heterocyclischen Ring einen anellierten Benzolring, der durch ein Radikal substituiert ist, das ausgewählt ist aus der Gruppe bestehend aus Carboxyl, (C₁-C₆)-Alkoxycarbonyl und 1-{[(Cyclohexyloxy)carbonyl]oxy}ethoxycarbonyl;
die eine Koppelreaktion einer Verbindung der Formel (II) nach einem der Ansprüche 1 - 11 umfasst, 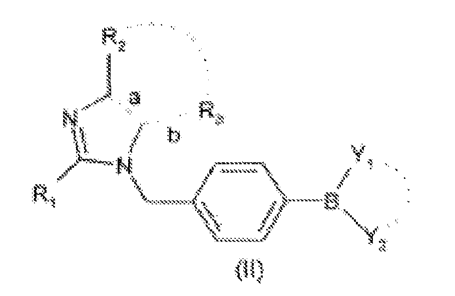 mit einer Verbindung der Formel (VII) 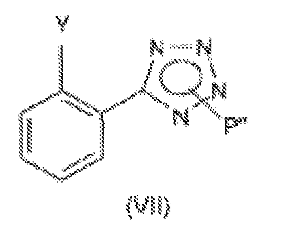
in einem geeigneten Lösungsmittelsystem und in Gegenwart von einer metallischen Verbindung und eine Basis;
worin P" ist H oder eine Schutzgruppe P', Y ist eine Abgangsgruppe und jede der Gruppen R₁, R₂ und R₃ ist eine Gruppe nach Anspruch 1 oder ein Zwischen- oder geschützte Form davon, die in eine solche Gruppe umgewandelt werden kann, und danach nach Bedarf die besagten Zwischen- oder geschützte Formen der Gruppen R₁, R₂ und R₃ in die Gruppen R₁, R₂ und R₃ nach Anspruch 1 umgewandelt werden; und optional wird, wenn P" eine Schutzgruppe P' ist, die erhaltene Verbindung einer Entschützungsreaktion unterzogen; und optional umfasst das Verfahren eine zusätzliche Etappe zur Umwandlung des Reaktionsprodukts in eine Salzform.

21. Der Vorbereitungsvorgang nach Anspruch 20, worin P" eine Schutzgruppe P' ist.

22. Der Vorbereitungsvorgang gemäß einem der Ansprüche 20 - 21, worin die Abgangsgruppe Y ist Br.

23. Der Vorbereitungsvorgang gemäß einem der Ansprüche 20 - 22, worin R₁ Butyl bedeutet, R₂ bilden einen spiro-anellierten Ring von fünf Kohlenstoffatomen mit dem Kohlenstoffatom des heterocyclischen Ringes, a ist eine einfache Verbindung, b ist eine doppelte Verbindung, R₃ ist 0 und P" ist H.

24. Der Vorbereitungsvorgang gemäß einem der Ansprüche 20 - 22, worin R₁ Butyl bedeutet, R₂ ist Cl, a ist eine doppelte Verbindung, b ist eine einfache Verbindung, R₃ bedeutet Hydroxymethyl und P" ist H.

25. Der Vorbereitungsvorgang gemäß einem der Ansprüche 20 - 22, worin R₁ Butyl bedeutet, R₂ ist Cl, a ist eine doppelte Verbindung, b ist eine einfache Verbindung, R₃ bedeutet Formyl und P" ist Trityl.

26. Der Vorbereitungsvorgang gemäß einem der Ansprüche 20 - 22, worin R₁ ein (C₁-C₄)-Alkoxylradikal ist; a ist eine doppelte Verbindung; R₂ und R₃ sind gebunden und bilden zusammen mit dem heterocyclischen Ring einen anellierten Benzolring, der durch ein Radikal substituiert ist, das ausgewählt ist aus der Gruppe bestehend aus Carboxyl, (C₁-C₆)-Alkoxycarbonyl und 1-{[(Cyclohexyloxy)carbonyl]oxy}-ethoxycarbonyl, und P" ist H oder Trityl.

27. Der Vorbereitungsvorgang nach Anspruch 26, worin R₁ ein Ethoxyradikal ist, R₂ und R₃ sind gebunden und bilden zusammen mit dem heterocyclischen Ring einen anellierten Benzolring, der durch ein Carboxylradikal substituiert ist, und P" ist H.

28. Der Vorbereitungsvorgang nach Anspruch 26, worin R₁ ein Ethoxyradikal ist, R₂ und R₃ sind gebunden und bilden zusammen mit dem heterocyclischen Ring einen anellierten Benzolring, der durch ein Ethoxycarbonyl-Radikal substituiert ist, und P" ist Trityl.

29. Der Vorbereitungsvorgang nach Anspruch 26, worin R₁ ein Ethoxyradikal ist, R₂ und R₃ sind gebunden und bilden zusammen mit dem heterocyclischen Ring einen anellierten Benzolring, der durch ein Methoxycarbonyl-Radikal substituiert ist, und P" ist Trityl.

30. Der Vorbereitungsvorgang nach Anspruch 26, worin R₁ ein Ethoxyradikal ist, R₂ und R₃ sind gebunden und bilden zusammen mit dem heterocyclischen Ring einen anellierten Benzolring, der durch ein 1-{[(Cyclohexyloxy)carbonyl]oxy}-ethoxycarbonyl-Radikal substituiert ist, und P" ist H.

31. Der Vorbereitungsvorgang gemäß einem der Ansprüche 20 -30, worin die metallische Verbindung aus Palladium, Nickel, einem Metallsalz und einem Metallkomplex ausgewählt ist.

32. Eine Verbindung der Formel (VI), 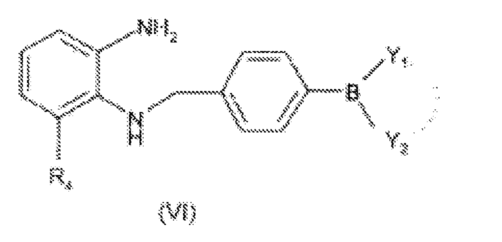 worin:
R₄ ein Radikal ist, das ausgewählt ist aus der Gruppe bestehend aus (C₁-C₆)-Alkoxycarbonyl und 1{[(Cyclohexyloxy)carbonyl)oxy}ethoxycarbonyl; Y₁ und Y₂ sind unabhängig voneinander ausgewählt aus der Gruppe bestehend aus Hydroxy, (C₁-C₄)-Alkoxy und Phenoxy, wobei letztere optional durch eine (C₁-C₄)-Alkoxy-, (C₁-C₄)-Alkyl- oder eine Halogengruppe substituiert ist, oder alternativ können Y₁ und Y₂ mit dem Boratom zusammen genommen werden, um eine zyklische Struktur zu bilden, die aus folgendem ausgewählt ist, 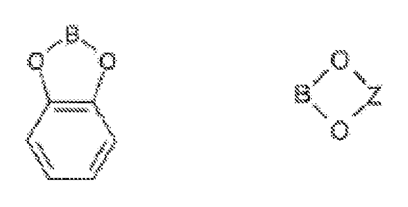
worin Z ausgewählt ist aus der Gruppe, bestehend aus (CH₂)n, (CH2)ᵣCRᵤRᵥ(CH₂)ₛ und CRᵤRᵥ(CH₂)ₜCRᵤRᵥ; n ist eine ganze Zahl von 2 bis 4, r und s sind ganze Zahlen von 0 bis 4, mit der Bedingung, dass r und s nicht beide 0 sind, t ist eine ganze Zahl von 0 bis 1, und Rᵤ und Rᵥ sind unabhängig voneinander ausgewählt aus der Gruppe bestehend aus H, (C₁-C₄)-Alkyl, Phenyl und mono- oder di-substituiertem Phenyl, wobei die Substituenten Halogen, (C₁-C₄)-Alkyl und (C₁-C₄)-Alkoxy sind; und jede der Gruppen R₄, Y₁ und Y₂ ist eine Gruppe nach der obigen Definition oder eine Zwischen- oder geschützte Form davon, die in eine solche Gruppe umgewandelt werden kann, und danach werden, wenn notwendig, die besagten Zwischen- oder geschützte Formen der Gruppen R₄, R₁, Y₁ und Y₂ in die Gruppen R₄, R₁, Y₁ und Y₂ nach der obigen Definition umgewandelt.

33. Ethyl 3-amino-2-{[(5,5-dimethyl-[1,3,2]dioxaborinan-2-yl)methyl]amino}benzoat.

## Revendications

1. Composé de formule (II), 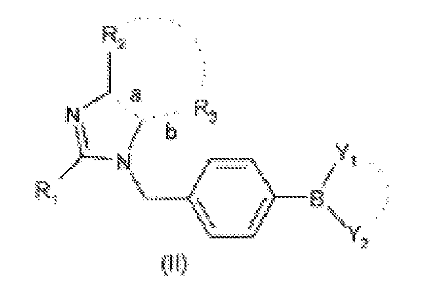 où:
Y₁, et Y₂ sont chacun choisis indépendamment à partir du groupe constitué d'hydroxy, (C₁-C₄)-alcoxy et phénoxy, ce dernier, optionnellement remplacé par un groupe (C₁-C₄)-alcoxy, (C₁-C₄)-alkyle ou halogène; ou alternativement Y₁, et Y₂ peuvent se joindre à l'atome de bore pour former une structure cyclique choisie parmi les suivantes, 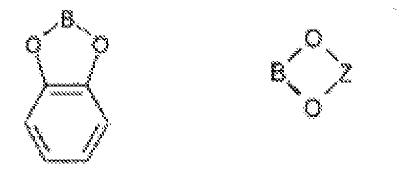 où Z est choisi parmi le groupe constitué de (CH₂)ₙ, (CH₂)ᵣCRᵤRᵥ(CH₂)ₛ et CRᵤRᵥ(CH₂)ₜCRᵤRᵥ; n est un nombre entier de 2 à 4, r et s sont des nombres entiers de 0 à 4 à condition que r et s ne soient pas tous les deux 0, t est un nombre entier de 0 à 1, et Rᵤ et Rᵥ sont choisis chacun indépendamment à partir du groupe constitué de H, (C₁-C₄)-alkyle, phényle et mono- ou di- phényle substitué, les substituants étant halogènes, (C₁-C₄)-alkyle et (C₁-C₄)-alkoxy,
a et b sont des liaisons simples ou doubles,
R₁ est un radical choisi à partir de (C₁-C₄)-alkyle et (C₁-C₄)-alkoxy:
R₂ est Cl, ou alternativement R₂ forme un anneau spiro-fondu de 4 à 6 atomes de carbone avec l'atome de carbone de l'anneau hétérocyclique; R₃ est 0 ou un radical choisi parmi le groupe constitué d'hydroxyméthyle et de formyle, qui sont optionnellement protégés; ou de manière alternative, R₂ et R₃ sont liés entre eux et forment avec l'anneau hétérocyclique un anneau benzénique fondu substitué par un radical choisi parmi le groupe constitué de (C₁-C₆)-alkoxycarbonyle et 1-[[(cyclohexyloxy)carbonyle]oxy]éthoxycarbonyle
à condition que
i) lorsque a est une liaison simple, b est une liaison double, R₂ forme un anneau spiro-fondu de 4 à 6 atomes de carbone avec l'atome de carbone de l'anneau hétérocyclique et R₃ est 0; et
(ii) lorsque a est une liaison double, b est une liaison simple, R₂ est Cl; et R₃, est un radical choisi parmi le groupe constitué d'hydroxyméthyle et de formyle, qui sont optionnellement protégés; ou de manière alternative, R₂ et R₃ sont liés entre eux et forment avec l'anneau hétérocyclique un anneau benzénique fondu, substitué par un radical choisi parmi le groupe constitué de (C₁-C₆)-akoxycarbonyle et 1-{[(cyclohéxyloxy)carbonyle]oxy}-éthoxycarbonyle.

2. Composé selon la revendication 1, où R₁ est butyle, R₂ forme un anneau spiro-fondu de 5 atomes de carbone avec l'atome de carbone de l'anneau hétérocyclique, a est une liaison simple, b est une liaison double et R₃ est 0.

3. Composé selon la revendication 1, où R₁ est butyle, R₂ est Cl, a est une liaison double, b est une liaison simple et R₃ est un radical hydroxyméthyle ou formyle.

4. Composé selon la revendication 1, où R₁ est un radical (C₁-C₄)-alkoxy, a est une liaison double; et R₂ et R₃ sont liés entre eux et forment avec l'anneau hétérocyclique un anneau benzénique fondu substitué par un radical choisi parmi le groupe constitué de (C₁-C₆)-akoxycarbonyle et 1-{[(cyclohéxyloxy)carbonyle]oxy}-éthoxycarbonyle.

5. Composé selon la revendication 4, où R₁ est un radical éthoxy, et R₂ et R₃ sont liés entre eux et forment avec l'anneau hétérocyclique un anneau benzénique fondu substitué par un radical éthoxycarbonyle.

6. Composé selon la revendication 4, où R₁ est un radical éthoxy, et R₂ et R₃ sont liés entre eux et forment avec l'anneau hétérocyclique un anneau benzénique fondu substitué par un radical méthoxycarbonyle,

7. Composé selon l'une quelconque des revendications 1-6, où Y₁ et Y₂ sont choisis indépendamment parmi le groupe constitué de: hydroxy, méthoxy, éthoxy et phénoxy, ou de manière alternative, Y₁ et Y₂ et l'atome de bore ensemble forment une structure cyclique, où Z est choisi parmi le groupe constitué de (CH₂)ᵣ,CRᵤRᵥ(CH₂)ₛ et CRᵤRᵥ(CH₂)ₜCRᵤRᵥ; r et s sont des nombres entiers de 0 à 4 à condition que r et s ne soient pas tous les deux 0; t est un nombre entier de 0 à 1 et Rᵤ et Rᵥ sont chacun choisis indépendamment à partir de méthyle et phényle.

8. Composé selon la revendication 7, où Y₁ et Y₂ sont hydroxy.

9. Composé selon la revendication 7, où Y₁ et Y₂ avec l'atome de bore forment ensemble une structure cyclique, où Z est CH₂C(CH₃)₂CH₂.

10. Composé selon la revendication 7, où Y₁ et Y₂ avec l'atome de bore forment ensemble une structure cyclique, où Z est C(CH₃)₂C(CH₃)₂.

11. Composé selon la revendication 1, qui est choisi parmi le groupe constitué de:
acide benzéneboronique 4-(2-n-butyle-4-chloro-5-formylimidazol-1-ylméthyle);
2-n-tutyle.-4-chloro-1-[4-(5,5-diméthyle-[1,3,2]dioxaborinane-2-yl)benzyle]-1 H-imidazole 5-carbaldéhyde;
acide benzénéboronique 4-(2-n-butyl-4-chloro-5-hydroxyméthylimidazol-1-yl méthyte);
2-n-hutyle-4-chloro-1-[4-(5,5-diméthyle-[1,3,2]dioxaborinane-2-yl)benzyle-1 H-imidazole-5-methanol;
acide benzénéboronique 4-(2-n-butyle-4-spirocyclopentane-2-imidazoline-5-one-1-ylméthyle);
2-n-butyle-3-[4-(5,5-diméthyle-[1,3,2]dioxahorinane-2-yl)benzyle]-1,3-diazaspiro[4.4]non-1-ene-4-one;
éthyle 1 -[(5,5-diméthyle-[1,3,2]dioxaborinane-2-yl)méthyle]-2-éthoxybenzim idazole-7-carboxylate;
méthyle 1 -[(5,5-diméthyle-[1,3,2]dioxaborinane-.2-yl)méthyle]-2-éthoxybenzimidazole-7-carboxylate; et
2-n-butyle-4-chloro-1-[4-(4,4,5,5-tetraméthyle-[1,3,2]dioxoborolane2-yl)benzyle]-1 H-imidazole-5-carbaldéhyde.

12. Procédé de préparation d'un composé de formule (H) tel que défini dans l'une quelconque des revendications 1 à 11, comprenant une réaction d'un composé de formule (III), 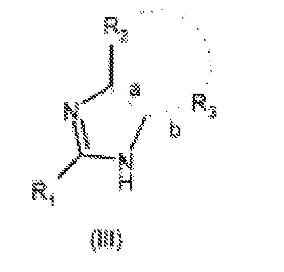 avec un composé de bore de formule (IV) 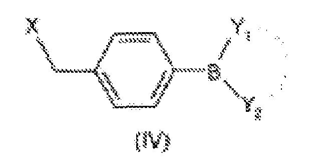 en présence d'une base et d'un solvant approprié:
où X est un groupe partant; et chaque R₁, R₂, R₃, Y₁ et Y₂ est un groupe tel que défini dans la revendication 1, ou une forme intermédiaire ou protégée de ceux-ci qui peut être transformée en un tel groupe, et successivement de transformation nécessaire desdites formes intermédiaires ou protégées des groupes R₁, R₂, R₃, Y₁ et Y₂ en groupes R₁, R₂, R₃, Y₁ et Y₂ tels que définis dans la revendication 1.

13. Procédé de préparation conformément à la revendication 12, où la base est une base inorganique choisie parmi le groupe constitué de hydride de métal alcalin, hydroxyde de métal alcalin, carbonate de métal alcalin et alkoxyde de métal alcalin.

14. Procédé de préparation selon l'une quelconque des revendications 12 à 13, où le groupe partant X est Br.

15. Procédé de préparation d'un composé tel que défini dans l'une quelconque des revendications 4 à 6, comprenant les phases de.
(a) réaction d'un composé de formule (V) 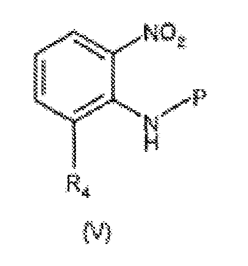 avec le composé de bore de formule (IV), 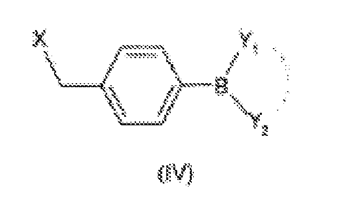 en présence d'une base et d'un solvant approprié; suivi par la segmentation du groupe amino-protecteur P et la réduction du groupe nitro afin de produire un composé de formule (VI); et 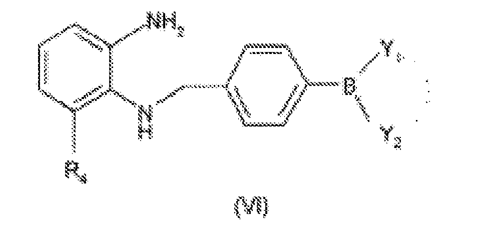
(b) traitement du composé (VI) avec un agent orthocarbonate d'alkyle ou agent carbonylant, afin de fournir un composé de formule (II'); 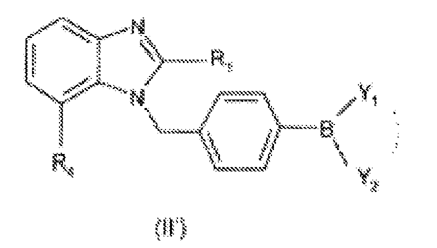
où P est un groupe amino-protecteur; X est un groupe partant; R₄ est un radical choisi parmi le groupe constitué de (C₁-C₆)-alkoxycarbonyle 1 {[(cyclohéxyloxy)carbonyle]oxy)-éthoxycarbonyle; Y₁ et Y₂ sont choisis chacun indépendamment parmi le groupe constitué d'hydroxy, (C₁-C₄)-alcoxy et phénoxy, ce dernier optionnellement substitué par un groupe (C₁-C₄)-alcoxy, (C₁-C₄)-alkyle ou halogène; ou de manière alternative Y₁ et Y₂ peuvent se joindre à l'atome de bore pour former ensemble une structure cyclique choisie parmi les suivantes, 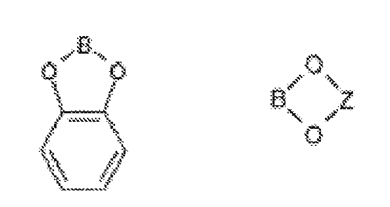 où Z est choisi parmi le groupe constitué de (CH₂)ₙ, (CH₂)ᵣCRᵤRᵥ(CH₂)ₛ et CRᵤRᵥ(CH₂)ₜCRᵤRᵥ; n est un nombre entier de 2 à 4; r et s sont des nombres entiers de 0 à 4 à condition que r et s ne soient pas tous les deux 0; t est un nombre entier de 0 à 1 et Rᵤ et Rᵥ sont choisis chacun indépendamment à partir du groupe constitué de H, (C₁-C₄)-alkyle, phényle et mono- ou di- phényle substitué, les substituants étant halogènes, (C₁-C₄)-alkyle et (C₁-C₄)-alkoxy, et chaque R₄, R₁, Y₁ et Y₂ est un groupe tel que défini ci-dessus, ou une forme intermédiaire ou protégée de ceux-ci qui peut être transformée en un tel groupe, et successivement une transformation nécessaire desdites formes intermédiaire ou protégée des groupes R₄, R₁, Y₁ et Y₂ en groupes R₄, R₁, Y₁ et Y₂ tels que définis ci-dessus.

16. Procédé de préparation selon la revendication 15, où Y₁ et Y₂ sont hydroxy.

17. Procédé de préparation selon la revendication 15, où Y₁ et Y₂ et l'atome de bore forment ensemble une structure cyclique, où Z est CH₂C(CH₃)₂CH₂.

18. Procédé de préparation selon la revendication 15, où Y₁ et Y₂ et l'atome de bore forment ensemble une structure cyclique, où Z est C(CH₃)₂C(CH₃)₂.

19. Procédé de préparation selon l'une quelconque des revendications 15-18 où le groupe partant X est Br.

20. Procédé de préparation d'un composé de formule (I), ou d'un sel pharmaceutique de celui-ci, 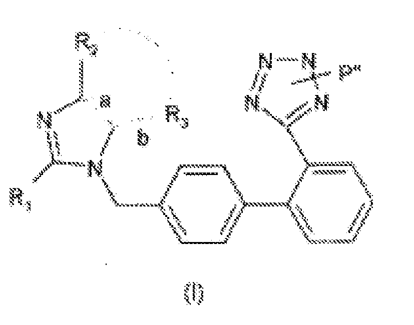 où:
a et b sont des liaisons simples ou doubles,
P" est H ou le groupe protecteur P';
R₁ est un radical choisi parmi (C₁-C₄)-alkyle et (C₁-C₄)-alkoxy:
R₂ est Cl; ou de façon alternative R₂ forme un anneau spiro-fondu de 4 à 6 atomes de carbone avec l'atome de carbone de l'anneau hétérocyclique; R₃ est 0 ou un radical choisi parmi le groupe constitué d'hydroxyméthyle et de formyle, qui sont optionnellement protégés; ou de façon alternative, R₂ et R₃ sont unis et forment ensemble avec l'anneau hétérocyclique, un anneau benzénique fondu substitué par un radical choisi parmi le groupe composé de carboxyle, (C₁-C₆)-alkoxycarbonyle et 1-{[(cyclohéxyloxy)carbonyle)oxy}éthoxycarbonyle;
à condition que:
(i) lorsque a est un liaison simple, b est une liaison double, R2 forme un anneau spiro-fondu de 4 à 6 atomes de carbone avec l'atome de carbone de l'anneau hétérocyclique et R₃ est 0. et
(ii) lorsque a est une liaison double, b est une liaison simple, R₁ est Cl: et R₃ un radical choisi parmi le groupe constitué d'hydroxyméthyle et de formyle, qui sont optionnellement protégés, ou de façon alternative, R₂ et R₃ sont unis et forment ensemble avec l'anneau hétérocyclique, un anneau benzénique fondu, substitué par un radical choisi parmi le groupe constitué de carboxyle, (C₁-C₆)-alkoxycarbonyle et 1-{[(cyclohéxyloxy)carbonyle]oxy}-éthoxycarbonyle,
lequel comprend une réaction de couplage d'un composé de formule (11), comme défini dans l'une quelconque des revendications 1-11, 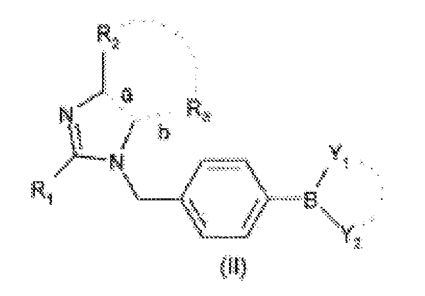 avec un composé de formule (VII) 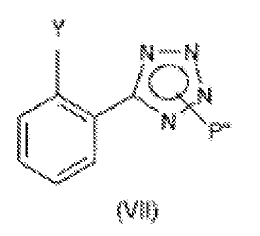 dans un système de solvant approprié et en présence d'un composé métallique et d'une base;
où P" est H ou un groupe protecteur P'; Y est un groupe partant: et chaque R₁, R₂ et R₃ est un groupe tel que défini dans la revendication 1, ou une forme intermédiaire ou protégée de celui-ci qui peut être transformée en un tel groupe, et successivement de transformation nécessaire des formes intermédiaire ou protégée des groupes R₁, R₂ et R₃ en groupes R₁, R₂ et R₃ tels que définis dans la revendication 1; et optionnellement lorsque P" est un groupe protecteur P' exposant le composé obtenu à une réaction de déprotection; et optionnellement le procédé inclut une étape supplémentaire de conversion du produit de réaction en forme de sel.

21. Procédé de préparation selon la revendication 20, où P" est un groupe protecteur P'.

22. Procédé de préparation selon l'une quelconque des revendications 20 à 21, où le groupe partant Y est Br.

23. Procédé de préparation selon l'une quelconque des revendications 20 à 22, où R₁ est butyle, R₂ forme un anneau spiro-fondu de cinq atomes de carbone avec l'atome de carbone de l'anneau hétérocyclique, a est une liaison simple, b est une liaison double, R₃ est 0 et P" est H.

24. Procédé de préparation selon l'une quelconque des revendications 20 à 22 où R₁ est butyle; R₂ est Cl, a est une liaison double, b est une liaison simple, R₃ est hydroxyméthyle et P" est H.

25. Procédé de préparation selon l'une quelconque des revendications 20 à 22 où R₁ est butyle, R₂ est Cl, a est une liaison double, b est une liaison simple, R₃ est formyle et P" est trityle.

26. Procédé de préparation selon l'une quelconque des revendications 20 à 22, où R₁ est un radical (C₁-_{C4})-alkoxyle, a est une liaison double; R₂ et R₃ sont unis et forment ensemble avec l'anneau hétérocyclique un anneau benzénique fondu substitué par un radical choisi parmi le groupe constitué de carboxyle, (C₁-_{C6})-alkoxycarbonyle et 1-{(cyclohexyloxy)carbonyle]oxy} -éthoxycarbonyle, et P" est H ou trityle.

27. Procédé de préparation selon la revendication 26, où R est un radical éthoxy, R₂ et R₃ sont unis et forment ensemble avec l'anneau hétérocyclique un anneau benzénique fondu substitué par un radical carboxyle, et P" est H.

28. Procédé de préparation selon la revendication 26, où R₁ est un radical éthoxy, R₂ et R₃ sont unis et forment ensemble avec l'anneau hétérocyclique un anneau benzénique fondu substitué par un radical éthoxycarbonyle, et P" est trityle

29. Procédé de préparation selon la revendication 26, où R₁ est un radical éthoxy, R₂ et R₃ sont unis et forment ensemble avec l'anneau heterocyclique un anneau benzénique fondu substitué par un radical méthoxycarbonyle, et P" est trityle,

30. Procédé de préparation selon la revendication 26, où R₁ est un radical éthoxy, R₂ et R₃ sont unis et forment ensemble avec l'anneau hétérocyclique un anneau benzénique fondu substitué par un radical 1-{(cyclohexyloxy)carbonyle]oxy} -éthoxycarbonyle, et P" est H.

31. Procédé de préparation selon l'une quelconque des revendications 20 à 30, où le composé métallique est choisi à partir de palladium, nickel, d'un sel métallique et d'un complexe métallique.

32. Composé de formule (VI), 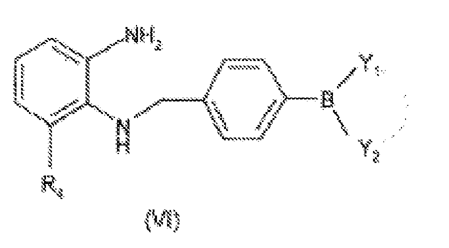 où:
R₄ est un radical choisi parmi le groupe constitué de (C₁-C₆)-alkoxycarbonyle et 1{[(cydohexyloxy)carbonyle)oxy}-éthoxycarhonyle; Y₁ et Y₂ sont choisis chacun indépendamment parmi le groupe constitué d'hydroxy, (C₁-C₄)-alcoxy et phénoxy, ce dernier optionnellement substitué par un groupe (C₁-C₄)-alcoxy, (C₁-C₄)-alkyle ou halogène; ou de façon alternative Y₁ et Y₂ peuvent être unis à l'atome de bore pour former ensemble une structure cyclique choisie parmi les suivantes, 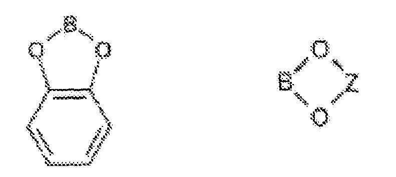
où Z est choisi parmi le groupe constitué de (CH₂)n, (CH2)ᵣCRᵤRᵥ(CH₂)ₛ et CRᵤRᵥ(CH₂)ₜCRᵤRᵥ; n est un nombre entier de 2 à 4; r et s sont des nombres entiers de 0 à 4 à condition que r et s ne soient pas tous les deux 0; t est un nombre entier de 0 à 1, et Rᵤ et Rᵥ sont choisis chacun indépendamment parmi le groupe constitué de H, (C₁-C₄)-alkyle, phényle et mono- ou di phényle substitué, les substituants étant halogènes (C₁-C₄₎-alkyle et (C₁-C₄)-alkoxy; et chaque R₄, Y₁ et Y₂ est un groupe tel que défini ci-dessus, ou une forme intermédiaire ou protégée de celui-ci qui peut être transformée en un tel groupe, et successivement de transformation nécessaire des formes intermédiaire ou protégée des groupes R₄, R₁, Y₁ et Y₂ en groupes R₄, R₁, Y₁ et Y₂ tels que définis ci-dessus.

33. Benzoate d'éthyle 3-amino-2- {[5,5-diméthyle-[1,3,2jdioxaborinane-2-yl)méthyle]amino}.
